# EUROPEAN PATENT APPLICATION

(11) **EP 1 619 500 A1**
(43) Date of publication of application: **25.01.2006**
(21) Application number: 04729231.3
(22) Date of filing: 23.04.2004
(51) Int. Cl.: G01N 33/53, G01N 37/00

(54) **BIOCHIP AND BIOCHIP KIT, AND METHOD OF PRODUCING THE SAME AND METHOD OF USING THE SAME**

(30) Priority: 25.04.2003 JP 2003122514; 23.10.2003 JP 2003363623
(71) Applicant: JSR Corporation, Tokyo 104-8410 (JP); OCTEC INC., Tokyo 160-0011 (JP)
(72) Inventor: OKUMURA, Katsuya, Tokyo 160-0011 (JP); MIHARA, Makoto, Tokyo 104-8410 (JP); YOSHIOKA, Mutsuhiko, Tokyo 104-8410 (JP)
(74) Representative: TBK-Patent
(86) International application number: PCT/JP2004/005878
(87) International publication number: WO 2004/097415

(57) **Abstract**

There are provided a biochip and a biochip kit, in which a target contained in an analyte is reacted with a probe with high efficiency in a short time, B/F separation efficiency is high, and high-sensitive quantitative determination and detection can be realized, and a production process thereof, and a method for reacting a target contained in an analyte with a probe, and, for example, separation and fractionation method and a detection and identification method for a target contained in an analyte, using the biochip kit. The biochip according to the present invention comprises a well(s) provided with a filter comprising straight pores, with a uniform pore diameter, provided at uniform pore spacings. A dispersion with probe-supported particles dispersed therein is contained in the well, and an analyte is placed in the well(s) to react the analyte with the probe-supported particles. A solution such as an analyte solution can be introduced into or discharged from the well through the filter.

## Description

### TECHNICAL FIELD

The present invention relates to a biochip comprising a well(s) having, at its bottom, a filter comprising straight pores with a uniform diameter, a biochip kit comprising said biochip and a vessel, a method for reacting or interacting a target, contained in an analyte which interacts with a probe-supported particle, with the probe, a method for B/F separation of a target from an analyte, a method for separating and fractionating a target from an analyte, and a method for detecting and identifying a reactive interaction between a target contained in an analyte and a bioprobe.

### BACKGROUND ART

For example, upon heating, double stranded DNA is brought to single stranded DNAs. Since these single stranded DNAs have complementary structures, they are mutually bound to each other. A Northern hybridization method has been established by taking advantage of this property. A method in which a fragment prepared by fragmenting DNA having a specific sequence having a proper length with a restriction enzyme or the like, or a synthesized oligonucleotide, is used as a probe for a search, for example, for a DNA fragment or an oligonucleotide having a sequence complementary to this fragment or oligonucleotide, has been carried out as a standard technique.

The Northern hybridization method, however, suffers from a problem that the procedure is complicated and even a small number of analytes cannot be treated in a short time. To overcome this problem, a DNA chip has been developed as a simple treatment method to which the Northern hybridization method is applied. In this DNA chip, the above oligonucleotide or the like is immobilized at high density on a substrate such as slide glass, and the analysis can be carried out in a short time. This DNA chip is currently widely used.

A typical example of this DNA chip comprises a oligonucleotide immobilized on a flat substrate such as silicon. In this chip, the length can be extended to about 20 to 30 bases by immobilizing one base onto a substrate and binding this base to other bases on a one base basis, and an oligonucleotide having a predetermined base sequence can be synthesized directly on the chip under light irradiation or in the presence of an acid to produce a DNA chip. For example, Japanese Patent Laid-Open No. 27000/1988 describes a DNA chip comprising an oligonucleotide or the like which has been synthesized in situ on a substrate such as silicon.

Alternatively, the DNA chip may be prepared by binding a nucleic acid such as cDNA or a previously synthesized oligonucleotide having a proper length onto a substrate. In this method, the so-called "Brown method" is used in which a separately provided nucleic acid is separated from a particulate carrier and is spotted onto a predetermined position by a spotter.

In the former method, however, since the base sequence is synthesized on the chip, even when the synthesis has failed only in a part of the base sequence, the whole chip is regarded as an unacceptable chip. As a result, the yield of the chip is y^{4np} wherein y is the probability of correct synthesis in one base synthesis step, n is the number of bases, and p is the number of probes. Accordingly, as the number of probes increases, the yield is geometrically lowered. To solve this problem, a spare probe is provided as a probe alternative to a defective probe. This means, however, disadvantageously results in increased substrate area.

On the other hand, in the latter method, the amount of the DNA spot and the position of the spot greatly depend upon the accuracy of the spotter and the like, and assuring good reproducibility is difficult.

Further, in the microarray or DNA chip, for one analyte, probes such as nucleic acids for several tens of thousands of items can be mounted, and an examination for several tens of thousands of different items can be simultaneously carried out. On the other hand, since the probe is immobilized on a substrate, the reaction of the analyte with the probe is a reaction between the immobilized probe and the analyte in the liquid, that is, a reaction known as the so-called "solid-liquid reaction." In general, the solid-liquid reaction is poor in reaction efficiency, and a few hours are necessary for the so-called hybridization reaction with the analyte.

Furthermore, in order to accurately detect the nucleic acid having a target base sequence, a nucleic acid having a sequence complementary to this nucleic acid (hereinafter often referred to as "probe") should be fixed in a narrow space with good efficiency. The number of probes which can be immobilized on the substrate is determined by the area occupied by individual probes and the size of the DNA chip per se, and immobilization of probes in number exceeding a certain number is physically impossible. Further, in chips of type which is immersed in the analyte for a reaction, such as currently used DNA chips, the size of the chip per se is preferably small because the amount of a usable analyte solution is sometimes very small.

When the size of the chip is reduced, however, the area where the probe can be immobilized is reduced and, consequently, due to the narrow space, the intensity of the detection signal is so weak that the detection sensitivity is lowered.

In order to eliminate the above drawback, in recent years, a chip comprising the above probe immobilized on three-dimensional gel has been produced and is commercially available (see, for example, Analytical Clinica Acta Vol. 444 p. 69 to 78 (2001)). In this chip, since the probe is three-dimensionally immobilized on three-dimensional gel and the probe density is high, the intensity of the detection signal is high, but on the other hand, since the signal intensity of noise is also high, the S/N ratio is not increased making it difficult to realize a significant improvement in detection accuracy. The reason why the signal intensity of noise is disadvantageously increased is believed to reside in that the removal of a product produced by a nonspecific reaction of the probe immobilized on the three-dimensional gel with the analyte is difficult.

On the other hand, a chip has also been developed in which hollow filaments are bundled, probes are bound onto the interior side wall of the hollow filaments to form a three-dimensional probe and thus to improve the probe density (see Japanese Patent Laid-Open No. 181074/2000).

Further, in recent years, a biochip has been developed in which a porous alumina substrate having vertical anisotropic pores is provided and probes are immobilized on the inner wall of the vertical pores to provide a three-dimensional structure and thus to increase the probe density, and, further, after a reaction of the analyte with the probe, a nontarget material can be washed away by taking advantage of a flow-down structure comprising vertically formed pores (see Published Japanese Translation of PCT Publication No. 504864/1997 (International Publication 01/12846)).

Even in all the above methods in which probes are bound to the interior side wall of hollow filaments or to the inner wall of pores in porous alumina, since the probes are immobilized on the fixed wall, in the reaction of the analyte with the probe, the analyte should approach or come into contact with the immobilized probe. In this case, however, the following points should be noted. The size of the probe is much smaller than the volume of the reaction space. When the probe is viewed on the basis of the scale of the reaction space, the probe is merely a projection slightly protruded from the fixed wall and, consequently, the probability that the analyte approaches or comes into contact with the probe is very low. Accordingly, these methods require stirring for a long period of time for the reaction.

Further, when probes are immobilized on the inner wall of the three-dimensional structure and the depth of the pore is increased, the surface tension of the side wall within the pore is increased and, thus, introduction/discharge of an analyte, a washing liquid and the like is difficult.

A method is also adopted in which, instead of the immobilization of probes on the fixed wall, probes are supported on particles, and, in a solution containing the probe-supported particles, the probe-supported particles are reacted with a target in the analyte. In this method, the probe-supported particles are three-dimensionally dispersed in the solution, and the probe-supported particles and the analyte can be mutually moved. Therefore, this method is advantageous in that the reactivity is very high. For example, a detection/identification method is known in which a target contained in an analyte is specifically reacted with a probe on the latex particle surface, and the contents of the target specifically reacted with and bound to the probe are analyzed by any B/F (bound form/free form) separation.

Nucleic Acid Research Vol. 14 p. 5037 to 5048 (1986) describes a method in which a target nucleic acid as an analyte is hybridized with a nucleic acid probe supported on a particle in a solution followed by centrifugation to remove the analyte remaining unreacted. To this day, this method is widely used. The centrifugation, however, disadvantageously requires a lot of time for separation, and, further, the separative power is not very high.

Japanese Patent Laid-Open No. 27000/1988 and Patent No. 2975603 disclose a method in which magnetic particles are used as particles for supporting probes and the magnetic particles are immobilized by a magnet for washing away the analyte remaining unreacted. At the present time, this method is also widely used. In order that the magnetic particles exhibit magnetic properties, however, in general, the size of the magnetic particles should be not less than 1 micron, and, since a metallic magnetic component such as ferrite is contained, the specific gravity is so high that sedimentation or agglomeration is likely to occur and, thus, care should be taken in storage and handling.

Japanese Patent Laid-Open No. 27000/1988 describes a method in which a target nucleic acid as an analyte is hybridized with a nucleic acid probe supported on a particle in a solution, and filtration and washing are then carried out through filter paper, followed by measurement of a reaction-derived signal concentrated on the filter paper. This method is also currently widely used. In this method, however, the amount of latex particles used as the particles is so large that a lot of time is required for the filtration, and, in addition, the filter paper is likely to be clogged.

In all the above methods using a probe-supported particle described in these documents, a multiplex assay or multiplex isolation fractionation using a plurality of probes cannot be carried out for one analyate.

A method in which particles provided with any identification label capable of identifying a plurality of probes are used has been proposed as a method for performing a multiplex assay using probe-supported particles. For example, U.S. Patent No. 5,736,330, Japanese Patent Laid-Open No. 81566/1987, and Japanese Patent Publication No. 54324/1995 describe methods in which a plurality of fluorescently colored particles or a plurality of particles different from each other in particle diameter are provided, different probes are supported on the respective particles, the type of the probe is specified by color, particle diameter or the like, and a reaction between the analyte and the probe-supported particle is detected by a flow cytometer. At the present time, this method is also widely used.

In this method, however, the probe-supported particle reacted with the analyte is filtered through a 96-hole plate with a filter, or B/F separation is carried out by centrifugation or the like, followed by dispensing in a flow cytometer for detection. In this case, the analyte is once treated in a plate with a filter or a centrifugal sedimentation tube for centrifugation and is then applied to the flow cytometer. This increases the number of steps and causes a fear of contamination with the plate or tube or a deposition loss of the analyte.

On the other hand, in the method using colors for identification, the number of types of identifiable colors is limited. Specifically, the number of types of light which can be dispersed by a combination of a certain wavelength range with light intensity is about 100. Accordingly, it is said that about 100 color/intensity combinations, that is, 100 probes, are the limit of simultaneous assays.

In addition, Japanese Patent Laid-Open No. 243997/1999 describes a method for identifying particles by taking advantage of particle size and shape. Further, Japanese Patent Laid-Open No. 346842/2000 describes a method in which particles are arranged one-dimensionally or two-dimensionally and, in order to partition probe particles different from each other in type, fine particles of different size are used as separation partition walls.

In all the methods described in the above documents in which particles are identified for multiplex assays, detection with a flow cytometer is carried out for the identification of particles. In the methods using the flow cytometer, however, particles are flowed into a capillary, and a laser beam is applied from the capillary in its transparent part to sequentially identify the particles. This disadvantageously requires a lot of time for detection. In this case, when the detection time should be shortened, a sufficient time should not be provided for the identification of one particle and, consequently, the detection accuracy is likely to be lowered. The capillary in the flow cytometer is expensive, and the replacement of the capillary every time when the analyte is replaced is difficult for cost reasons. To avoid this difficulty, the capillary should be cleaned every time when the analyte is replaced. This further requires a time for thorough cleaning.

Biochips include DNA chips in which a DNA fragment or an oligonucleotide has been immobilized as the probe and protein chips in which a protein such as an antigen or an antibody or a chemical compound capable of specifically reacting or interacting with the protein has been immobilized. These protein chips and the like also involve the above problems.

The DNA chips and protein chips can be applied through various probes immobilized onto the substrate to functional analyses of genes possessed by organisms; clinical assays for confirming affected conditions of diseases such as various infectious diseases; gene polymorphic analyses; selection of therapeutic drugs according to the gene sequence of a patient, called "tailor-made therapy"; screening of chemical compounds or proteins for the development of pharmaceutical preparations, or toxic screening of chemical compounds; and other various types of screening and the like.

Currently used biochips, however, do not have satisfactory detection sensitivity for use in the above various applications. For example, in order to find out a disease in a very early stage, a very small amount of disease-derived marker which comes out from cells to the outside the cells in a very early stage of the disease should be detected. It is said that the detection sensitivity of the existing biochips is unsatisfactory for the detection of the very small amount of marker. Accordingly, the development of a biochip having higher sensitivity has been desired.

The use of a single probe is unsatisfactory for obtaining accurate information about conditions of a disease of a patient to minimize a diagnostic error, and a simultaneous multi-item assay using a plurality of different probes are preferred. Accordingly, the development of a biochip which can perform a multiplex assay with high detection sensitivity has been desired.

The present invention has been made with a view to solving the above problems of the prior art, and an object of the present invention is to provide a biochip in which
· a target contained in an analyte is reacted with a probe with high efficiency in a short time,
· B/F separation efficiency is high, and
· high-sensitive detection and identification can be realized, and

to provide a process for producing the same.

Another object of the present invention is to provide a biochip kit which can realize direct transfer of a liquid between wells, specifically between biochips each provided with a plurality of wells, or between a biochip provided with a plurality of wells and a vessel provided with a plurality of wells.

A further object of the present invention is to provide
· a method for separating and fractionating one or more targets from one analyte using said biochip,
· a method for separating and fractionating one or more targets from a number of analytes in a simultaneous parallel manner,
· a method for assaying one analyte for one or more targets, and
· a method for assaying a number of analytes in a simultaneous parallel manner for one or more targets.

### DISCLOSURE OF INVENTION

(i) A biochip characterized by comprising a well(s) having, at its bottom, a filter comprising straight pores with a uniform diameter arranged at uniform pore spacings.
(ii) The biochip according to the above item (i), characterized in that said filter has a thickness of 1 to 10 µm.
(iii) The biochip according to the above item (i) or (ii), characterized in that the open area ratio of the filter is 15 to 60%.
(iv) The biochip according to any one of the above items (i) to (iii), characterized in that the surface of the filter is formed of silica, titania, or alumina.
(v) The biochip according to any one of the above items (i) to (iv), characterized by comprising a plurality of said wells provided integrally with each other.
(vi) The biochip according to any one of the above items (i) to (iv), characterized in that said well is singularly provided.
(vii) The biochip according to any one of the above items (i) to (vi), characterized in that a reinforcing rib part is provided on the upper side or lower side of said filter in said well.
(viii) The biochip according to the above item (vii), characterized in that said reinforcing rib part is of an integral type provided with a plurality of through-holes.
(xi) The biochip according to the above item (vii) or (viii), characterized in that said reinforcing rib part is joined directly to said filter.
(x) The biochip according to the above item (vii) or (viii), characterized in that said reinforcing rib part is formed so as to continuously extend from said filter, said reinforcing rib part and said filter being formed of an identical material.
(xi) The biochip according to any one of the above items (i) to (x), characterized in that a nonporous part free from pores of said filter is provided on the bottom of said well in a predetermined width from the periphery of said well.
(xii) The biochip according to any one of the above items (i) to (xi), characterized in that a first filter is provided at the bottom of the well(s) and a second filter is provided on the side opposite to the first filter so that the well(s) is sandwiched between said first and second filters.
(xiii) The biochip according to any one of the above items (i) to (xii), characterized in that a dispersion with a probe-supported particle dispersed therein is placed in said well(s).
(xiv) The biochip characterized in that the ratio between the diameter of said particle and the pore diameter of said filter is particle diameter/pore diameter = 1.1 to 2.5, and said particle diameter and said pore spacing satisfy a relationship represented by formula: particle diameter < pore spacing < particle diameter x 10.
(xv) The biochip characterized in that the diameter of said particle and the pore diameter and pore spacing of said filter satisfy a relationship represented by formula: particle diameter > pore diameter + pore spacing/2.
(xvi) The biochip according to the above item (xiii), characterized in that said well contains a dispersion in which probe-supported particles having at least one identification means for providing probe identification information have been dispersed.
(xvii) The biochip according to the above item (xvi), characterized in that said identification means is at least one means selected from color, shape, diameter and gene sequence in said probe-supported particle.
(xviii) The biochip according to the above item (xvi) or (xvii), characterized in that a plurality of probe-supported particles which are identical to each other in probe identification information in all of said identification means are contained in an identical well and said wells are identical to each other in said probe identification information for a plurality of probe-supported particles contained therein.
(xix) The biochip according to the above item (xvi) or (xvii), characterized in that a plurality of probe-supported particles which are identical to each other in probe identification information in all of said identification means are contained in an identical well and said wells are different from each other in said probe identification information for a plurality of probe-supported particles contained therein.
(xx) The biochip according to the above item (xvi) or (xvii), characterized in that a plurality of probe-supported particles which are different from each other in probe identification information in said at least one identification means are contained in an identical well and said wells are identical to each other in construction of said probe identification information in all the identification means for a plurality of probe-supported particles contained therein.
(xxi) The biochip according to the above item (xvi) or (xvii), characterized in that a plurality of probe-supported particles which are different from each other in probe identification information in said at least one identification means are contained in an identical well and said wells are different from each other in construction of said probe identification information in at least one of said identification means for a plurality of probe-supported particles contained therein.
(xxii) A biochip kit characterized by comprising: a vessel; and, a plurality of wells formed integrally with each other or a single well in the biochip according to any of the above items (i) to (xxi) housed in or connected to said vessel.
(xxiii) The biochip kit according to the above item (xxii), characterized in that said vessel is formed integrally with said well(s).
(xxiv) The biochip kit according to the above item (xxii), characterized in that said vessel is formed independently of said well(s).
(xxv) The biochip kit according to any of the above items (xxii) to (xxiv), characterized in that said vessel is provided with well(s) corresponding to said well(s) in said biochip.
(xxvi) The biochip kit according to the above item (xxv), characterized in that a through-hole is provided at the bottom of said well(s) in said vessel.
(xxvii) The biochip kit according to the above item (xxv) or (xxvi), characterized in that said biochip and said vessel are connected to each other so that the corresponding wells are connected to each other.
(xxviii) The biochip kit according to the above items (xxii) to (xxvii), characterized in that said vessel comprises a plurality of plates stacked on top of each other, said plurality of plates being selected from plates with a through-hole and plates free from a through-hole.
(xxix) A biochip kit characterized by comprising a plurality of biochips according to any of the above items (i) to (xxi) which are connected to each other so that the corresponding wells are connected to each other.
(xxx) The biochip kit according to any of the above items (xxii) to (xxix), characterized in that the flat part provided on the lower end of the well side part in said biochip is connected directly to the flat part provided on the upper end of the well side part in said separate vessel or said separate biochip so that the wells are connected to each other.
(xxxi) The biochip kit according to any of the above items (xxii) to (xxix), characterized in that either a positioning concave part into which a convex part provided on the upper end of the well side part in said separate vessel or said separate biochip is to be fitted, or a positioning convex part into which a concave part provided on the upper end of the well side part in said separate vessel or said separate biochip is to be fitted is provided on the lower end of the well side part in said biochip.
(xxxii) A process for producing a biochip according to any of the above items (i) to (xxi), characterized by comprising: providing a plate having a structure of at least two layers different from each other in composition of a material constituting the layer; subjecting said plate to pattern etching from its one side to the boundary between the two layers to form a well hole; and subjecting said plate to pattern etching from its other side to the boundary between the two layers to form filter pores, thereby preparing a biochip comprising a well and a filter connected to each other.
(xxxiii) A process for producing a biochip according to any of the above items (i) to (xxi), characterized in that silicon wafers are etched to prepare a filter, a rib, and a well which are then stacked on top of each other.
(xxxiv) A method for operating a biochip kit, characterized in that, in a biochip kit according to any of the above items (xxii) to (xxxi) comprising said vessel and said biochip, said vessel being provided independently of said well(s) in said biochip, a solution is placed in said vessel and said well(s) in said biochip is vertically moved in said solution to bring said solution in said vessel into contact with said probe-supported particle and/or solution within said well(s).
(xxxv) A method for operating a biochip kit, characterized in that the interface of a solution contained in said vessel in a biochip kit according to any of the above items (xxii) to (xxxi) is vertically moved to bring said solution in said vessel into contact with said probe-supported particle and/or solution within said well(s).
(xxxvi) A method for operating a biochip kit, characterized in that, in a biochip kit according to any of the above items (xxii) to (xxxi) comprising said vessel for housing said biochip therein, a pressure differential is created between said vessel and said chip or between mutual wells in said chip to cause contact of a liquid with said probe-supported particle within said well(s), transfer of a liquid between wells, or both of them.
(xxxvii) A method for operating a biochip kit, characterized in that, in a biochip kit according to any of the above items (xxii) to (xxxi) comprising said vessel connected to said biochip, a pressure differential is created between said vessel and said chip or between mutual wells in said chip to cause contact of a liquid with said probe-supported particle within said well(s), transfer of a liquid between wells, or both of them.
(xxxviii) The method for operating a biochip kit according to any of the above items (xxxiv) to (xxxvii), characterized in that the solution within said vessel is brought into contact with said probe-supported particle and/or solution within said well to perform mixing, diffusion, reaction, separation, or washing of contents within said biochip.
(xxxix) The method for operating a biochip kit according to any of the above items (xxxiv) to (xxxviii), characterized in that an identical analyte is introduced into each well in said biochip.
(xl) The method for operating a biochip kit according to any of the above items (xxxiv) to (xxxviii), characterized in that analytes introduced into respective wells in said biochip are different from each other.
(xli) A method for reacting a target contained in an analyte with a probe, characterized by comprising the steps of:
   placing a specific particle in said wells of said biochip in a kit according to any of the above items (xxii) to (xxxi) to constitute a chip according to any of the above items (xviii) to (xxi);
   introducing an analyte-containing solution into said wells of said biochip to bring the system to such a state that said analyte can come into contact with said particles within all the wells; and
   vertically moving said wells in said solution contained in the vessel of said biochip, or vertically moving the interface of said solution contained in the vessel of said biochip, or applying a differential pressure to circulate said solution present within or outside said wells to react said target contained in said analyte with said probe.
(xlii) A method for B/F separation of a target from an analyte, characterized by comprising the steps of:
   placing specific particles in said wells of said biochip in a kit according to any of the above items (xxii) to (xxxi) to constitute a chip according to any of the above items (xviii) to (xxii);
   introducing an analyte-containing solution into said wells of said biochip to bring the system to such a state that said analyte can come into contact with said particles within all the wells;
   lowering the height of the interface of said solution until the position of the interface of said solution is below the lower surface of said filter at the bottom of said well to remove said analyte remaining unreacted with the probe supported on the particle from within each of said wells; and
   introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing.
(xliii) A method for fractionally isolating a target in an analyte, characterized by comprising the steps of:
   placing a specific particle in said wells of said biochip in a kit according to the above items (xxx) or (xxxi) to constitute a chip according to any of the above items (xviii) to (xxi);
   introducing an analyte-containing solution into said wells of said biochip to bring the system to such a state that said analyte can come into contact with said particle within all the wells;
   lowering the height of the interface of said solution until the position of the interface of said solution is below the lower surface of said filter at the bottom of said well to remove said analyte remaining unreacted with the probe supported on the particle from within each of said wells;
   introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing; and
   fitting a concave part, a convex part, or a smooth part, provided on the lower end of the well side part of said biochip, and a convex part, a concave part, or a smooth part, corresponding to the concave part, convex part, or smooth part in said biochip, provided on the upper end of said vessel, together, and then introducing a separating agent solution into said wells of said biochip, whereby said target in said analyte is isolated from said particle and is transferred to said wells of said vessel.
(xliv) A method for detecting and identifying an interaction between a target contained in an analyte and a probe, characterized by comprising the steps of:
   placing specific particles in said wells of said biochip in a kit according to any of the above items (xxii) to (xxxi) to constitute a chip according to any of the above items (xviii) to (xxi);
   introducing an analyte-containing solution into said wells of said biochip to bring the system to such a state that said analyte can come into contact with said particles within all the wells;
   lowering the height of the interface of said solution until the position of the interface of said solution is below the lower surface of said filter at the bottom of said well to remove said analyte remaining unreacted with the probe supported on the particle from within each of said wells;
   introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing;
   positioning said particles within said wells on said pores in said filter; and
   detecting and identifying a reaction or an interaction between the probe supported on said particle and the target in said analyte.
(xlv) The method for detecting and identifying an interaction between a target contained in an analyte and a probe according to the above item (xliv), characterized in that, for each particle, both probe indentification information of said particle and information about a reaction or interaction between said probe supported on said particle and said target contained in said analyte are detected.
(xlvi) The method for detecting and identifying an interaction between a target contained in an analyte and a probe according to the above item (xliv), characterized in that, for said particles in each well, information about identification of said probe supported on said particle is identified, and the state of an interaction between said probe and said target contained in said analyte is then measured to calculate information about an interaction for each well based on information about the state of interaction for each particle.

The present invention provides a biochip in which
· a target contained in an analyte is reacted with a probe with high efficiency in a short time,
· B/F separation efficiency is high, and
· high-sensitive quantitative determination and detection can be realized, and

further provides a process for producing the same.

Further, according to the present invention, there is provided a biochip kit which can realize direct transfer of a liquid between wells, specifically between biochips each provided with a plurality of wells, or between a biochip provided with a plurality of wells and a vessel provided with a plurality of wells.

Furthermore, according to the present invention, there are provided
· a method for isolating and fractionating one or more targets from one analyte,
· a method for isolating and fractionating one or more targets from a number of analytes in a simultaneous parallel manner,
· a method for assaying one analyte for one or more targets, and
· a method for assaying a number of analytes in a simultaneous parallel manner for one or more targets,

said methods each using said biochip.

### BRIEF DESCRIPTION OF THE DRAWINGS

Fig. 1 is a typical cross-sectional view showing a bottom side of a well provided in a biochip in one embodiment of the present invention;
Fig. 2 is a typical cross-sectional view showing an example of pores in a filter;
Fig. 3 is a typical cross-sectional view of a bottom side of a well provided with a reinforcing rib part;
Fig. 4 is a typical top view showing a reinforcing rib part provided on a filter surface;
Fig. 5 is an electron photomicrograph of a well bottom part having a reinforcing rib part provided on a filter surface;
Fig. 6 is a typical cross-sectional view showing a bottom side of a well provided with a reinforcing rib part;
Fig. 7 is a typical cross-sectional view showing a bottom side of a well provided with a reinforcing rib part;
Fig. 8 is a typical cross-sectional view showing a bottom side of a well provided with a concave part for registration in a reinforcing rib part;
Fig. 9 is a typical cross-sectional view showing a bottom side of a well provided with a concave part for registration in a reinforcing rib part;
Fig. 10 is a typical cross-sectional view showing a bottom side of a well having a pore-free part in a predetermined width from the periphery of the well on the upper surface of a bottom part in the well;
Fig. 11 is a diagram illustrating a method for preparing a rib or a well using an optical molding;
Fig. 12 is a top view of a biochip in one embodiment of the present invention and a cross-sectional view taken on line A-A';
Fig. 13 is a top view of a biochip in one embodiment of the present invention and a cross-sectional view taken on line A-A';
Fig. 14 is a cross-sectional view of a biochip comprising a first filter and a second filter in combination;
Fig. 15 is a cross-sectional view illustrating a method for preparing a biochip kit comprising a biochip connected to a vessel;
Fig. 16 is a cross-sectional view showing an example in which a smooth and transparent plate for use in optical detection is provided in a plate as a bottom part or a lid part in a vessel of a biochip kit;
Fig. 17 is a cross-sectional view showing an example of a biochip kit comprising a vessel formed using a plate;
Fig. 18 is a cross-sectional view showing an example of a biochip kit comprising chips in a multistage;
Fig. 19 is a diagram illustrating a method for preparing a chip comprising a plurality of wells;
Fig. 20 is a diagram illustrating a method for operating a biochip according to the present invention;
Fig. 21 is a cross-sectional view of a chip illustrating one step in a separation/fractionation method and detection method for a target in an analyte according to the present invention;
Fig. 22 is a cross-sectional view of a chip illustrating one step in a separation/fractionation method and detection method for a target in an analyte according to the present invention;
Fig. 23 is a cross-sectional view of a chip illustrating one step in a separation/fractionation method for a target in an analyte according to the present invention;
Fig. 24 is a cross-sectional view of a biochip kit having fine through-holes in a vessel at its well bottom face;
Fig. 25 is a cross-sectional view of a chip illustrating one step in a detection method for a target in an analyte according to the present invention;
Fig. 26 is a diagram illustrating an example of a biochip according to the present invention in which probe-supported particles are contained in a well(s);
Fig. 27 is a diagram illustrating a method for introducing an analyte in a well of a biochip according to the present invention;
Fig. 28 is an electron photomicrograph showing probe-supported particles located on filter pores;
Fig. 29 is an electron photomicrograph showing an image of the bottom part of each well in a chip;
Fig. 30 is a graph showing a relationship between the total discharge amount and the discharge time in the case where a bovine serum dilute solution is flowed into a filter in a chip in Example 3 and was discharged from a lower lid in a lower vessel; and
Fig. 31 is a graph showing the results of a test performed in the same manner as in Example 3 under a differential pressure of 18 gf/cm² for eight combinations different from each other in chip pore diameter and pore spacings.

### BEST MODE FOR CARRYING OUT THE INVENTION

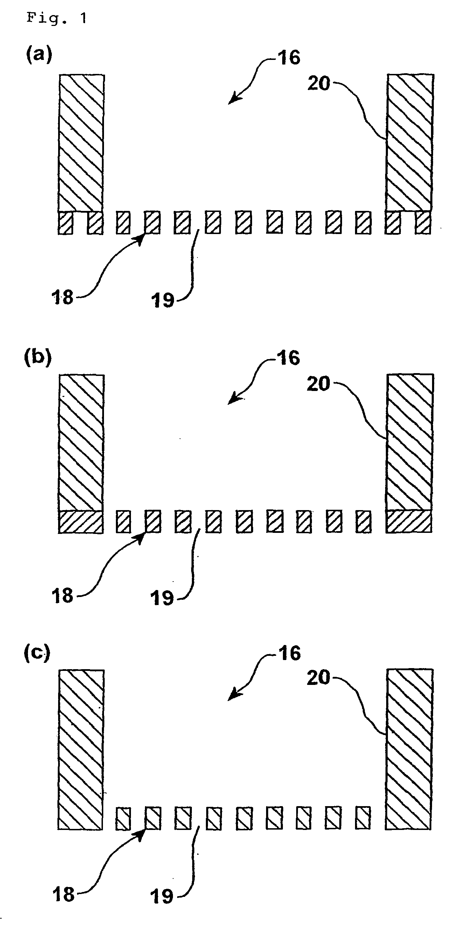

The present invention will be described in more detail with reference to the accompanying drawings. Fig. 1 is a typical cross-sectional view of a bottom side of a well provided in biochip in one embodiment of the present invention.

As shown in the drawing, a filter 18 having pores 19 is provided in a well 16 at its bottom. This filter 18 is constructed so that a liquid such as an analyte or a medium with an analyte dissolved or dispersed therein is passed through the filter while probe-supported particles which interact with an analyte, contained in the well 16 are not discharged to the outside of the well.

In the present invention, straight pores having a uniform diameter are provided at uniform pore spacings in the filter 18. The "uniform pore diameter" as used herein means that the error of pore diameter is not more than 20%, preferably not more than 10%, in terms of CV (coefficient of variation) value. Pores with a pore diameter error of not more than 20% in terms of CV value can be prepared by the method which will be described later, and the difference in dimension between probe-supported particles contained in the well 16 and the pore diameter can be made small.

A variation in pore diameters in conventional membrane filters or the like is about 10 times. For example, when a filter with a pore diameter of 0.2 µm is used, the diameter of particles which can reliably filtered is about 5 µm. By contrast, when the filter with a uniform pore diameter is used, the diameter of the pores in the filter is very uniform and, thus, the difference between the particle diameter and the pores can be reduced. Therefore, the pore diameter can be further increased, the filtration capability of the filter can be improved, and the filtration pressure can be reduced.

In the filter with a uniform pore diameter, the pore diameter is not particularly limited. In general, however, the pore diameter is 0.01 µm to 100 µm, preferably 0.1 µm to 50 µm, more preferably 0.5 µm to 15 µm.

The term "uniform pore spacings" as used herein means that the error of the pore spacings is not more than 15% in terms of CV (coefficient of variation) value. The pore spacing is not particularly limited. However, when the pore spacing is excessively small, the strength of the filter is low. On the other hand, when the pore spacing is excessively large, the open area ratio is lowered. Accordingly, the pore spacing is generally twice or less the pore diameter, preferably 0.5 µm to 10 µm. The term "pore spacing" as used herein refers to the shortest distance in the pore-free part located between adjacent pores.

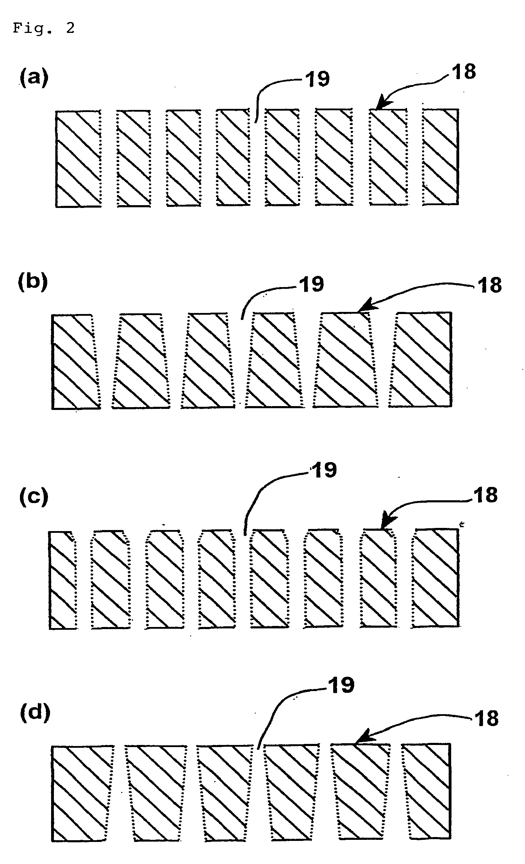

Further, the "straight" pores as used herein means that pores are formed without halfway branching. For example, the pore may be such that a perpendicular line drawn from the center of an opening formed on one filter surface to the other filter surface departs from a perpendicular line drawn from the center of an opening formed on this other filter surface to the one filter surface, because this departing does not cause a significant difference in pressure loss. Further, the pores may be such that the perpendicular lines do not substantially depart from each other and, at the same time, the pore diameter on the primary side (upper surface side) of the filter is different from the pore diameter on the secondary side (lower surface side). Examples of such pore shapes are shown in Figs. 2 (a) to (d). The shape of the section of the pore perpendicular to the pore extended direction is not particularly limited and may be any of a cylindrical column, a quadrangular pyramid, a polyangular pyramid and the like. The shape of the section, however, is preferably one having an obtuse angle or circular from the viewpoint of minimizing the formation of a meniscus. When the volume of the well is not less than a predetermined value, for example, not less than 0.1 microliter, the meniscus poses no severe problem, and the shape of the section may be, for example, a quadrangular prism or a quadrangular pyramid.

The adoption of such straight pores minimizes the length of pores formed in the filter and, thus, reduces the area of contact with the pore wall and can minimize transfer resistance under pressure in the filtration.

Further, even when the probe-supported particles are accumulated on the primary side of the filter and, consequently, clogging occurs, the particles do not enter the inside of the filter and stay on the surface of the filter (that is, a completely clogged model is formed). Therefore, in this case, the particles can easily be dispersed from the filter surface toward the primary side by flushing from the secondary side of the filter, and the clogging of the filter can be eliminated again to regenerate the filter, leading to the prolongation of the service life of the filter.

Thus, when straight pores having a uniform diameter are formed in the filter at uniform pore spacings, probe-supported particles are arranged on the opening on the primary side, and, if necessary, a liquid such as an analyte can be flushed from the secondary side to disperse the particles toward the primary side. Therefore, in this case, a reaction of the analyte with probe-supported particles and detection can be easily carried out.

Further, when the discharge of the probe-supported particles on the secondary side of the filter is not permitted and 100% of the particles should be trapped, 100% of the particles can be trapped without lowering permeability coefficient and filtration efficiency by taking into consideration, for example, particle size in such a manner that the pore diameter of the filter is made larger than the minimum diameter of the particles.

In conventional filter paper and membrane filter, the pore diameter and the pore spacing are not uniform, and the pores per se have a three-dimensionally complicated structure, and pores which are finer than the pores on the surface of the filer are present within the filter. Therefore, a part of the particles contained in the filtrate is trapped in the pores present within the filter, and, consequently, clogging called "intermediate clogging model" occurs. In eliminating this clogging, when a liquid is fed from the secondary side to take out the particles from within the filter to the primary side, the flushing effect is not satisfactory and all the particles cannot be taken out from the filter to the primary side. As a result, the particles are confined within the filter, and the amount of the particles used in the detection is reduced. Further, the particle capture efficiency of the filter is probabilistic, and, in order to provide a capture efficiency of 100%, a filter satisfying "minimum particle diameter with particle diameter distribution as a sieving object > maximum diameter of filter diameter distribution" should be selected. This results in lowered permeability coefficient and filtration efficiency.

In the present invention, the thickness of the filter is preferably 1 to 10 µm, more preferably 2 to 7 µm. When the thickness of the filter is larger than 10 µm, the filtration resistance during the filtration of the liquid is large, while, when the thickness of the filter is less than 1 µm, the mechanical strength of the filter is unsatisfactory.

In the present invention, the open area ratio of the filter is preferably 15 to 60%, more preferably 20 to 50%. When the open area ratio is less than 15%, the filtration efficiency is lowered, while, when the open area ratio is more than 60%, the mechanical strength of the filter is unsatisfactory.

The filter may be in various forms having pores. Specific examples thereof include filters formed by pressing by a mold, filters formed by forming pores in a woven fabric or a film by applying a laser beam or a neutron beam, filters formed by scratching a resin or metallic thin film and expanding pores through the action of tension, filters formed by forming pores in a base material by photoetching, and filters formed by resin molding.

The above-described filter with pores having a uniform pore diameter and formed at even pore spacings may be formed, for example, by photolithography, specifically by coating a resist onto a predetermined organic or inorganic film and then conducting pattern etching to form predetermined pores. In order to ensure film thickness having predetermined mechanical strength and predetermined pore diameter, high-aspect etching is necessary and may be carried out by anisotropic etching.

Further, the filter may also be prepared by a method using an expanded metal. For example, cuts are formed in a 30 µm-thick stainless steel foil in a staggered form in a mold and are expanded to form rhomboidal through-holes. This method provides a filter with a maximum distance of the rhomboidal pore of 30 µm and an open area ratio of about 60%. Subsequently, the expanded metal filter is further pressed by a convex mold to form a predetermined concave face, whereby a plurality of wells which have been integrally formed are formed. Alternatively, a method may also be adopted in which a group of wells, for example, in a honeycomb or round form in which openings are vertically extended from the upper surface to the lower surface are separately prepared using a resin or a metal, a thermoplastic resin solution is coated onto the bottom face of the group of wells, and the coating is dried to heat bond the heated filter and wells to each other, whereby the expanded metal filter and the group of wells are bonded to form wells.

Further, as described below, a method may also be adopted in which the well side part and the filter are integrally molded with a resin.

According to a particularly preferred production process of a biochip, for a plate comprising a plurality of materials different from each other in composition, for example, aluminum/alumina, metallic silicon/silica, or metallic titanium/titania, pattern etching is carried out from both sides of the plate to form a filter and a well. Specifically, for a metal oxide layer formed of, for example, alumina, silica, or titania, photoetching is carried out to the boundary between the metal oxide layer and the metal layer to form a filter, and, subsequently, for the metal layer formed of, for example, aluminum, metallic silicon, or metallic titanium, photoetching is carried out to the boundary between the metal layer and the metal oxide layer, whereby a biochip comprising a well and a filter joined to each other can be prepared.

According to this method, the use of a multilayer material comprising a metal and a metal oxide or the like which have been previously integrated with each other can realize the preparation of an assembly comprising a filter and a well which have been integrally joined to each other. This assembly has no fear of causing liquid leakage from the interface of the filter and the well.

Further, since a transparent material such as alumina, silica, or titania may be used for filter layer formation, when optical detection is carried out, the behavior of the particles can be observed directly through the filter layer.

For example, from the viewpoint of reducing filtration resistance, the material for filter formation is preferably a material having a high level of affinity for a solution received in the well, specifically a hydrophilic material in the case where the solution is aqueous, and an oleophilic material in the case where the solution is oily.

Hydrophilic organic materials include, for example, polyethylene vinyl resins, crosslinked polyvinyl alcohol resins, polyglycol acid resins, polyamide resins, polyimide, cellulose acetate resins, triacetyl cellulose, cellulose nitrate resins, epoxy resins, and copolymers of various acrylates, for example, a copolymer of 2-methacryloyloxyethyl phosphorylcholine with methacrylate.

Oleophilic organic materials include, for example, polyethylenes, polypropylenes, polystyrenes, polymethyl methacrylate resins, liquid crystal polymers, polycarbonates, polyamide resins, polyimides, polyethylene terephthalates, polyethylene naphthalates, cycloolefins, polymethylpentenes, polyarylates, polysulfones, and polyethersulfones.

Inorganic materials include, for example, metals, such as iron, nickel, copper, zinc, aluminum, silicon, titanium, tantalum, magnesium, molybdenum, tungsten, rhodium, palladium, silver, gold, platinum, stainless steel, brass, red brass, bronze, phosphor bronze, aluminum-copper alloy, aluminum-magnesium alloy, aluminum-magnesium-silicon alloy, aluminum-zinc-magnesium-copper alloy, and iron-nickel alloy; metal oxides, such as silica, alumina, titania, zirconia, and tantalum oxide; metal nitrides, such as SiN, TiN, and TaN; metal carbides, such as SiC and WC; carbon materials, such as diamond, graphite, and diamond like carbon (DLC); and glass such as soda glass, borosilicate glass, Pyrex (trademark) glass, and quartz glass.

The surface of the oleophilic material may be subjected to plasma treatment, corona treatment, ion treatment or the like to form hydroxyl or carboxyl groups. Further, a hydrophilic metal or metal oxide may be formed on the surface, for example, by plating. Alternatively, a hydrophilic material, for example, a copolymer of 2-methacryloyloxyethyl phosphorylcholine with methacrylate, or a polyethylene glycol derivative, may be coated. Alternatively, after coating of glycidyl methacrylate, the epoxy group may be decyclized.

Among the above materials, materials having a surface formed of alumina, silica, or titania are particularly preferred as the filter material. Since these are hydrophilic, nonspecific adsorption of a bioanalyte is on a low level, and, further, an aqueous analyte or a washing liquid easily enters into the filter. When not only the surface but also the internal part of the filter is formed of the above material, the filter is transparent. This is advantageous in that, when optical detection is adopted, a reaction or interaction between the particulate probe and the analyte marker can be easily detected and identified through the transparent filter.

When the filter is formed using alumina, silica or titania as the surface material, a method may be adopted in which a chip comprising a filter and a well is formed of aluminum, metallic silicon, or metallic titanium, and oxidation is then carried out for conversion to alumina, silica, or titania. Alternatively, in this case, a method may be adopted in which a plate formed of aluminum/alumina, metallic silicon/silica, or metallic titanium/titania may be provided and is used for the formation of a well and a filter.

### <Well part>

The number of wells provided in one biochip is not particularly limited and may be determined, for example, by the type of the probe-supported particle or the number of probe-supported particles to be received, and the chip may have either a single well or a plurality of wells which have been integrally formed with each other. Even when only one type of probe-supported particle is used and, at the same time, a number of particles for separation and purification or other purposes are received in the well, a construction may be adopted in which a plurality of wells are provided and identical probe particles are dispersed and placed in respective wells.

As shown in Figs. 1 (a) and 1 (b), the well may be formed of a material which is different from the material constituting the filter. Alternatively, as shown in Fig. 1 (c), the well may be formed integrally with the filter using a substantially identical material. In Figs. 1 (a) and 1 (b), the filter material is different from the well material. A method may be adopted in which the filter and the well are formed separately from each other using an identical material and the filter may be then joined to the well.

As shown in Figs. 1 (b) and 1 (c), from the viewpoint of mechanical strength, the well is preferably connected to the filter so that the well is jointed to the filter layer in its part where no filter is formed.

The diameter and height of the well part are not particularly limited and may be properly determined by taking into consideration the number of necessary particles to be received and the reaction volume. When the diameter of the well is large, the area of the filter face is also large. In this case, the filter is likely to be deformed or destructed by the total pressure applied to the filter. However, the deformation or destruction can be prevented by providing a rib which will be described later.

In a chip having a plurality of wells, these wells are not always required to have an identical diameter, and wells having a different diameter may be provided while taking into consideration the number of particles to be received or the reaction volume.

The shape of the well hole defined by the well side part is not particularly limited. Preferably, however, the well hole is in an obtuse-angle or circular form from the viewpoint of minimizing the meniscus formation.

### <Reinforcing rib part>

In the well having a filter at its bottom as described above, since the thickness of the filer is small, when the hole diameter of the well is large or when the open area ratio of the filter is large, the strength of the well bottom part is unsatisfactory and, thus, the filter is likely to be broken or damaged, for example, during use of the filter. To overcome this problem, preferably, a rib-like reinforcing means is provided on the upper surface side or lower surface side of the filter to reinforce the well bottom part.

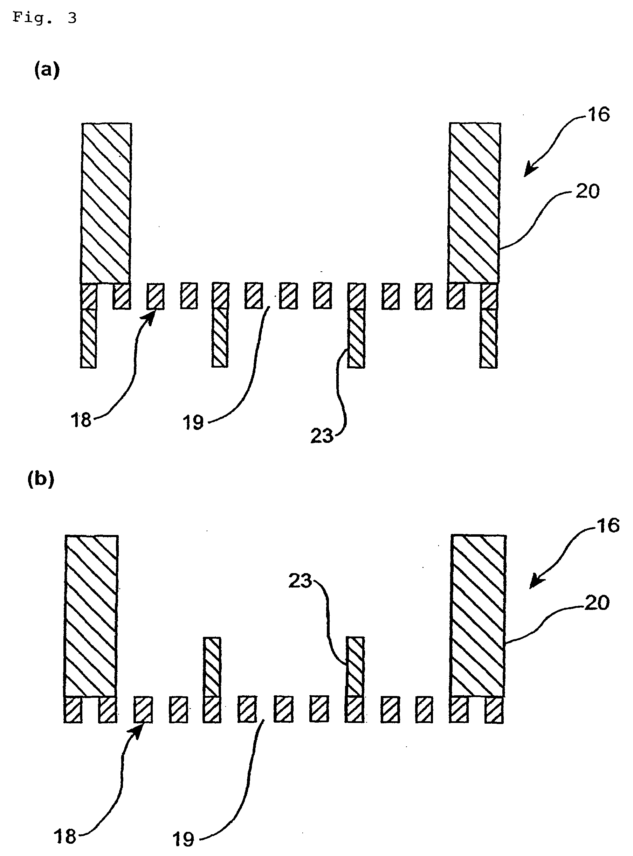

Fig. 3 is a typical cross-sectional view of the bottom side of the well provided with the reinforcing rib part. In Fig. 3 (a), a rib part 23 having a projected strip site is provided on the lower surface side of the filter 18. In Fig. 3 (b), a rib part 23 is provided on the upper surface side of the filter 18.

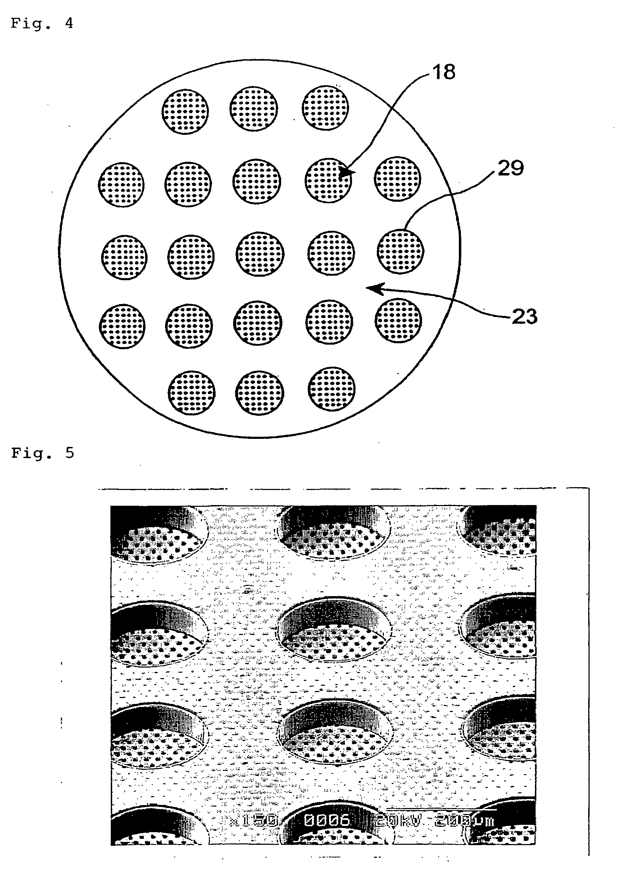

Fig. 4 is a typical top view of an assembly comprising a reinforcing rib part 23 provided on the surface of the filter 18. As shown in the drawing, preferably, the reinforcing rib part 23 is in an integral form having a plurality of through-holes 29 with parts between through-holes 29 being contiguous to each other. The form of the through-hole 29 in its section substantially perpendicular to the through-hole 29 extended direction is not particularly limited and may be, for example, in any of a circular form, a rectangular form, and a hexagonal or other polygonal form. Preferably, however, the form of the through-hole 29 in its section substantially perpendicular to the through-hole 29 extended direction is in a circular form or a hexagonal or other obtuse-angle polygonal form from the viewpoint of reducing the meniscus phenomenon. Fig. 5 is an electron photomicrograph of a well bottom part where a reinforcing rib part has been actually provided on the filter.

The height of the rib in the reinforcing rib part is preferably 10 µm to 2000 µm, more preferably 100 µm to 1000 µm, although it depends upon the bottom area of the well and the material of the rib.

The provision of the reinforcing rib part can improve the mechanical strength of the filter, and, thus, a large chip area can be realized. Further, even when the filter is thin, the resistance to the mechanical pressure can be provided and, at the same time, the depth of filter pores may be small. Therefore, the filtration pressure can be further lowered.

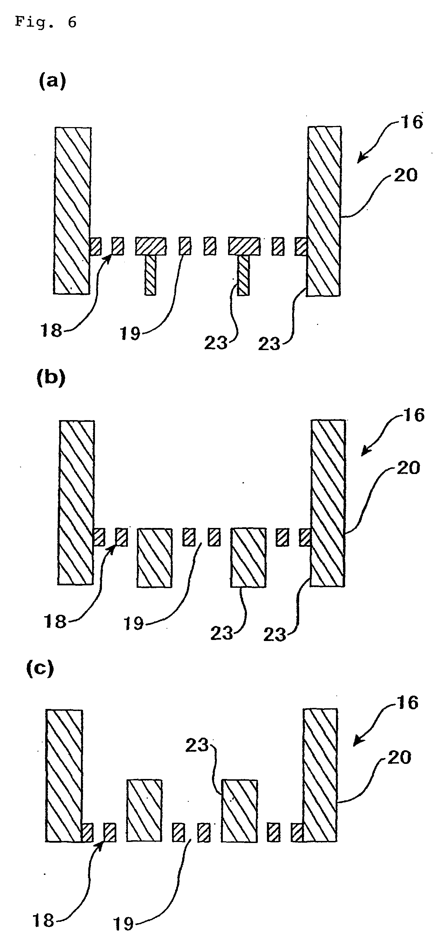

The reinforcing rib part may be joined by a method in which, as shown in Fig. 6 (a), a reinforcing rib part is formed separately from the filter and the reinforcing rib part and the filter are then joined to each other, or by a method in which, as shown in Figs. 6 (b) and 6 (c), the pore of the filter 18 is filled by collapsing a rib or well material by an additive method or the like for joining. Regarding the side wall 20 of the well, the side wall is extended ahead of the filter 18, and the lower end part of the side wall may be utilized as a part of the reinforcing rib 23.

When a separately formed reinforcing rib part or well side wall is joined to the filter, this joining is preferably carried out by direct joining without use of any adhesive. The direct joining may be carried out by a method which will be described later, for example, a method in which a filter and a reinforcing rib part or a well side wall are stacked on top of each other by lamination.

In this method, direct joining is carried out without an adhesive and the like, and, thus, problems caused in the filtration can be eliminated including separation of the interface of the reinforcing rib part or the well side wall and the filter, or the entry of the liquid to be filtered into the interface. In particular, in the case of a chip where the diameter of the opening in the wells is small and the number of wells is large, joining between the well side wall and the filter becomes difficult geometrically, often leading to liquid leakage at the interface. Direct joining between the well and the filter without the aid of any adhesive can eliminate the fear of liquid leakage. The reinforcing rib part is installed as a mechanical reinforcing material for the filter, and, thus, when the bonding between the reinforcing rib part and the filter is unsatisfactory, an improvement in mechanical strength cannot be expected.

However, direct joining without the aid of any adhesive can render the filter and the rib integral with each other with satisfactory strength, contributing to improved mechanical strength.

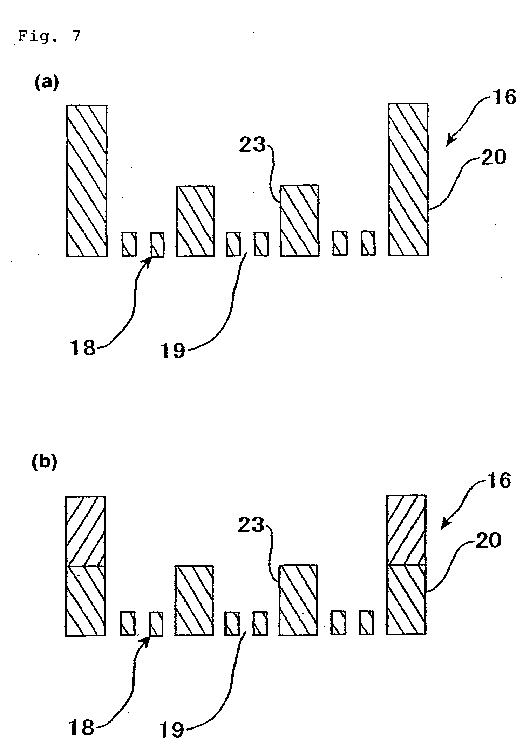

As shown in Figs. 7 (a) and 7 (b), the reinforcing rib part and the filter can be formed using an identical material by continuous molding. In this case, since the reinforcing rib part and the filter are formed integrally with each other, this is a preferred embodiment from the viewpoint of strength. Here the fact that the original material is identical suffices for the identical material. For example, a silicon silica material prepared by oxidizing a part of metallic silicon to convert the metallic silicon to silicon oxide, or a silicon/SiC or silicon/SiN material prepared by carbonizing or nitriding a part of metallic silicon to convert the metallic silicon to SiC or SiN is also regarded as an identical material. Likewise, the side wall of the well and the filter can also be formed by continuous molding.

This can be carried out by any method which will be described later, for example, by a method in which, in previously preparing a filter, hole formation is not carried out in the well and rib part and, in subsequent formation of the well and rib, registration and the well and rib formation are carried out, that is, the filter and the reinforcing rib part or the well side wall are prepared from an identical material, for example, by machining or etching.

Further, the filter 18 in its rib 23 part or well side wall 20 part may be if necessary free from pores. Specifically, a method may be adopted in which, as shown in Figs. 1 (b) and 1 (c), at the time of the preparation of the filter 18, a pore-free part is previously formed and the rib 23 or the well side wall 20 is positioned and formed on the pore-free part, or alternatively, a method may be adopted in which, when the rib 23 and the filter 18 are prepared by an additive method, plugging is simultaneously carried out.

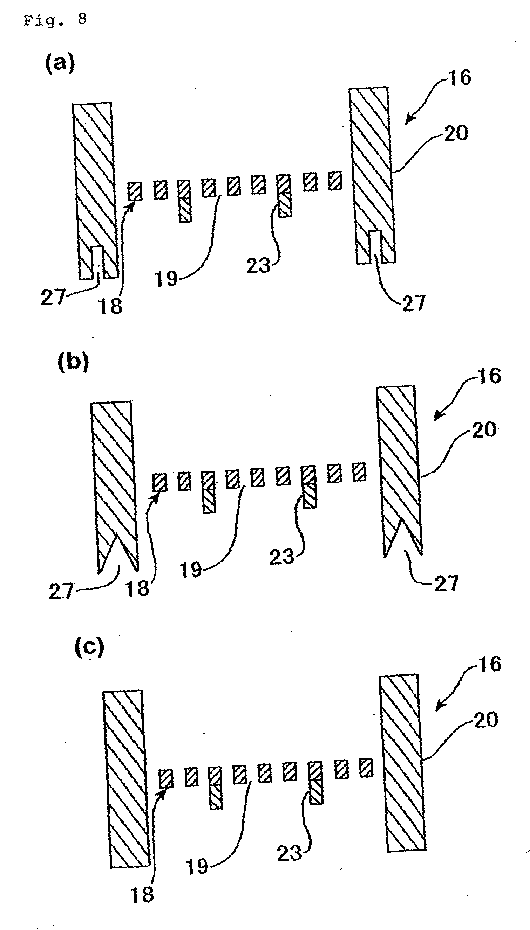

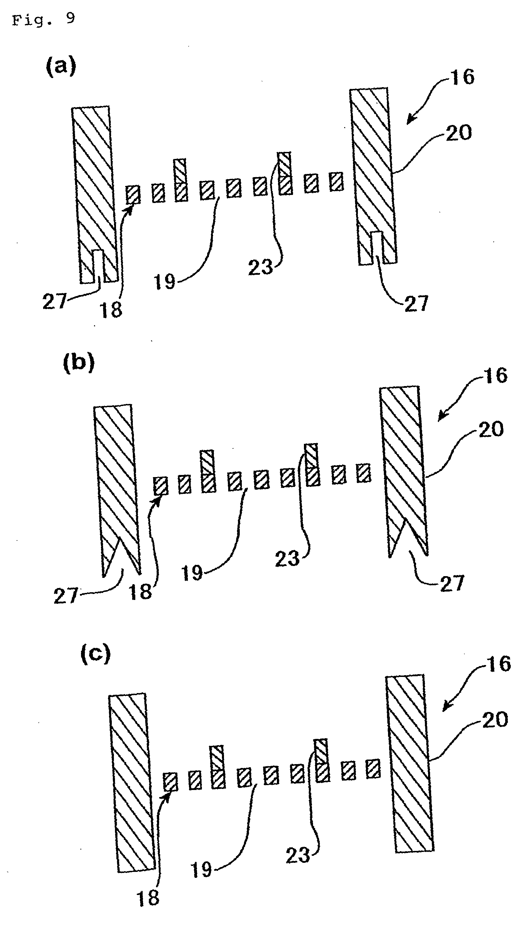

In wells shown in Figs. 8 (a) and 8 (b) and Figs. 9 (a) and 9 (b), the reinforcing rib part 23 is provided on the lower surface or upper surface of the filter and, in addition, a registration concave part 27 into which a convex part provided in a separate vessel is fitted is provided at a position on the lower end side of the well side part 20 in the reinforcing rib part 23. The concave part 27 may specifically have any proper shape depending upon the convex part provided in the separate vessel. Specifically, the concave part 27 provided in the well is a groove in a female part as a connector for the vessel at the time of the transfer of the solution within a predetermined well into a predetermined well in another vessel, and, by fitting the vessel in its male part into the female part, the solution within the well can be transferred without leakage to a part other than the vessel in its predetermined part. The shape of the groove in the concave part for registration is not particularly limited so far as the convex part as the male part provided on the vessel can be fitted into the female part.

Alternatively, without providing any concave or convex part for registration of the well, as shown in Fig. 8 (c) and Fig. 9 (c), the bottom face may be made smooth without forming the concave part 27 for joining. The higher the level of smoothness in the smooth face, the stronger the joining between faces. For example, when a commercially available silicon wafer for semiconductor, particularly a very smooth silicon wafer, is used, a very strong smooth joint surface can be provided.

In the case where a large number of wells containing a solution to be transferred and a vessel provided with a number of corresponding wells are present, the transfer of a liquid by the well having the above concave part for registration is effective in transferring solutions in all the corresponding wells to the corresponding wells in a simultaneous parallel manner. This further effective in the transfer of solutions from a vessel comprising a plurality of wells to a chip comprising the corresponding wells, or in the transfer of solutions between a plurality of chips.

The rib provided with a groove can be prepared in the same manner as adopted in the conventional rib by various production processes which will be described later. The width and shape of the well side part are not particularly limited. Preferably, however, the width of the well side part is not less than 100 µm, more preferably not less than 200 µm and not more than 10 mm, and more preferably not less than 300 µm and not more than 5 mm. When the width of the well side part is less than 100 µm, the convex part in the vessel cannot be fitted into the concave part for registration without difficulties, or in joining between smooth faces, the joint area is reduced by misregistration. When the width of the well side part exceeds 10 mm, the proportion of the well part in a predetermined area is excessively small and, consequently, the efficiency of chip preparation is lowered.

Independently of whether or not the concave part for registration is a part of the reinforcing rib 23, the concave part is formed at the lower end of the side part 20 of the well. Conversely, the concave part formed at the lower end of the well may be in a convex form or smooth. In this case, for fitting, a concave part or a smooth part should be formed in the corresponding vessel or chip.

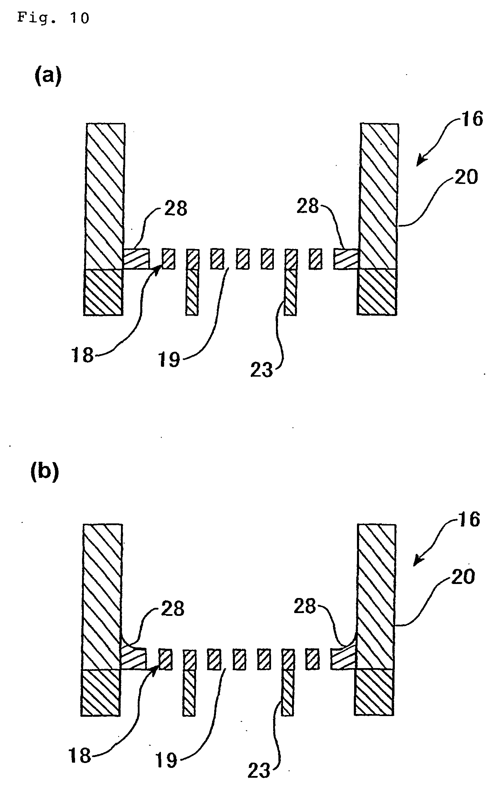

In the well shown in Fig. 10, a pore-free part 28 where the pore 19 in the filter 18 is not formed is provided on the upper surface of the bottom part of the well in a predetermined width from the periphery of the well.

The predetermined width of the pore-free part 28 is preferably 5 µm to 50 µm, more preferably 10 µm to 30 µm, as measured from the periphery of the well bottom part. In particular, as shown in Fig. 10 (b), the surface of the pore-free part 28 is preferably inclined. When the pore-free part is not provided and the pore is provided to the edge of the wall of the rib or the well, in some cases, unfavorable phenomena such as a stay of the particle in the edge of the wall or further accumulation of the particle on the edge of the wall occur. The provision of the pore-free part increases the mechanical strength in the connection part between the well and the filter and the fear of causing separation between the well and the filter is reduced. Further, the solution causes a laminar flow which causes the solution to be transferred from the edge of the wall in the well or rib toward the center part, and, thus, the stay or accumulation of the particles on the edge of the wall in the well or rib can be prevented. Further, in the optical detection which will be described later, since no particle is present at the edge of the wall, the inhibition of the detection of the particle at the edge of the wall by light reflection from the well wall can be prevented.

The well having this pore-free part may be prepared, for example, by a method in which a filter having a pore-free zone in a specific region is previously prepared and the well or the reinforcing rib is brought in register with and joined to the part of the pore-free zone, or by a method in which the well or the reinforcing rib is stacked onto the pore-free part, for example, by an additive method. Further, as shown in Fig. 10 (b), the structure in which the surface of the pore-free part 28 is inclined can be formed by previously forming a filter part and then wetetching the well in the direction of the height of the well to cause an etching residue.

Various production processes of the above well will be explained.

The first method is to stack the filter or the like by electroplating. In this method, an electrically conductively treated substrate is previously provided, and patterning is carried out on the substrate using a resist or the like, and, while protecting parts not to be electroplated by the resist, current is allowed to flow across the substrate and a plating solution to form a metal or ionic polymer material on the substrate only in its predetermined parts.

An electrically conductively treated substrate is first provided, and a resist is then coated thereon. After the formation of the resist film on the electrically conductive substrate, a photomask is provided, and UV light is used for exposure and development to form a post of the resist as a reversal pattern of the filter on the electrically conductive sheet. In these methods, the pattern limit depends upon the resolution of the resist. However, for example, when THB-110N (tradename: manufactured by JSR corporation) is used, a resist post having a pattern pitch of 5 µm and an aspect ratio of 2 can be prepared.

Next, a metal or an ionic resin is electrically filled into between the posts by electroplating, that is, by allowing alternating or direct current to flow. Metal materials which can be filled by electroplating include gold, nickel, copper, iron, and iron-nickel alloy. For example, a nickel sulfamate bath (a mixed solution composed of 700 g/liter 60% sulfamic acid, 5 g/liter nickel bromide, and 35 g/liter boric acid, bath temperature 50°C) may be used as a plating solution for nickel electroforming. For example, a copper sulfate bath (a mixed solution composed of 200 g/liter copper sulfate, 60 g/liter sulfuric acid, and 30 mg/liter chlorine ion, bath temperature 30°C) may be used as a copper electroplating solution. For example, an iron sulfamate bath (a mixed solution composed of 400 g/liter iron sulfamate, 30 g/liter ammonium sulfamate, and 100 mg/liter formalin, bath temperature 46°C) may be used as an iron electroplating solution. For example, in the case of the nickel sulfamate bath, a 5 µm-thick electroplated product can be provided by allowing a direct current to flow under conditions of voltage 6 V and current density 3A/dm² for about 10 to 20 min.

Resin materials which can be filled by electroplating include epoxy resins, acrylic resins, and polyimide resins. These resins include, for example, acrylic resins, for example, those manufactured by Shimizu Ekote Co., Ltd. and POWERTOP U manufactured by Nippon Paint Co., Ltd.

In any of the metals and resins, various additives may be added. For example, the chemical properties of the formed film can be varied by adding, for example, fine particles of metal oxides such as silica, titania, and alumina, and fine particles of fluororesins.

Subsequently, in the case of resin electroforming, finally, heating is carried out at about 200°C to cure the resin and, at the same time, to fire and remove the resist. In the case of electroplating, the resist may be removed, for example, by a resist stripper THB-S1 (tradename: manufactured by JSR corporation).

After the preparation of the filter by the above method, the well or the reinforcing rib part can be formed in the same manner as in the formation of the filter and further by electroplating. Specifically, in the same manner as described above, a resist film is formed on the filter, followed by photoetching and filling of an electroplating material. In this case, in general, since the height of the well is larger than the thickness of the filter, the resist film is thick. For this reason, the resist is preferably used in such a state that a dry film resist is stacked. In general, a plated product having an aspect between the line width and the thickness exceeds 2 cannot be formed by a single plated product formation procedure without difficulties. In this case, photoetching and electroplating for plated product formation are repeated a few times to provide a well having a predetermined height.

In the second method, at least one of the filter, the well, and the reinforcing rib part is prepared by etching. The object to be etched is not particularly limited to metals, metal oxides, organic materials and the like so far as the filter can be formed. However, mechanically strong materials are particularly preferred, and specific examples thereof include: engineering plastics, such as liquid crystal polymers, polycarbonates, polyamide resins, polyimides, polyethylene terephthalates, polyethylene naphthalates, polyallylates, polysulfones, and polyethersulfones; metals such as iron, nickel, copper, zinc, aluminum, silicon, titanium, tantalum, magnesium, molybdenum, tungsten, rhodium, palladium, silver, gold, platinum, stainless steel, brass, red brass, bronze, phosphor bronze, aluminum-copper alloy, aluminum-magnesium alloy, aluminum-magnesium-silicon alloy, aluminum-zinc-magnesium-copper alloy, and iron-nickel alloy; metal oxides, such as silica, alumina, titania, zirconia, and tantalum oxide; metal nitrides, such as SiN, TiN, and TaN; metal carbides, such as SiC and WC; carbon materials, such as diamond, graphite, and diamond like carbon (DLC); and glass such as soda glass, borosilicate glass, Pyrex (trademark) glass, and quartz glass.

Etching can be carried out by a conventional method. However, it should be noted that, when etching is carried out in an excessively high aspect ratio, there is a fear of causing nonuniform pore diameter and shape. The aspect ratio provided by conventional chemical etching is substantially not more than 5, preferably not more than 3. However, a filter having a good filtration property can be realized by using a filter having an actively formed reverse tapered shape in this aspect range.

When the well or rib shape is prepared by etching, the aspect ratio is preferably not less than 5. An aspect of not less than 5 can be achieved by anisotropic etching of the material. For example, in the case of a single crystal material such as silicon, great crystal dependency upon an aqueous KOH solution, ethylenediamine pyrocatechol (EDP), tetramethyl ammonium hydroxide (TMAH) or other etching solution is utilized. In the case of silicon, the etching rate of (111) plane is much lower than other crystal planes, and etching with an aspect of about 100 can be realized by conducting chemical etching using a silicon wafer having a surface with (110) plane in such a state that a side having an opening in the mask material is matched with the direction of (111) plane. These methods are described in "Nano Sukeru Kako Gijutsu (Nano scale processing technique)," edited and written by The Japan Society for Precision Engineering, The Nikkan Kogyo Shimbun, Ltd. (1993).

If necessary, reactive ion etching (RIE) using plasma can be carried out. For example, anisotropic etching perpendicular to a substrate can be carried out by gas containing SF₆ and Freon-based chlorine gas. These methods are described in "'02 Saishin Handoutai Purosesu Gijutsu ('02 Advanced semiconductor process technique)", Press Journal Inc. (2001).

In particular, for a plate comprising a plurality of materials different from each other in composition, for example, aluminum/alumina, metallic silicon/silica, or metallic titanium/titania, pattern etching is preferably carried out from both sides of the plate to form a filter and a well. Specifically, for a metal oxide layer formed of, for example, alumina, silica, or titania, photoetching is carried out to the boundary between the metal oxide layer and the metal layer to form a filter, and, subsequently, for the metal layer formed of, for example, aluminum, metallic silicon, or metallic titanium, photoetching is carried out to the boundary between the metal layer and the metal oxide layer, whereby a biochip comprising a well and a filter joined to each other can be prepared.

According to this method, the use of a multilayer material comprising a metal and a metal oxide or the like which have been previously integrated with each other can realize the preparation of an assembly comprising a filter and a well which have been integrally joined to each other. This assembly has no fear of causing, for example, liquid leakage from the interface of the filter and the well.

Further, since a transparent material such as alumina, silica, or titania may be used for filter layer formation, when optical detection is carried out, the behavior of the particles can be observed directly through the filter layer.

Such multilayer materials include, for example, an aluminum/alumina plate prepared by oxidizing the surface of an aluminum plate to alumina having a predetermined thickness, metallic titanium/titania prepared by oxidizing metallic titanium in the same manner as described above, a silicon wafer with an oxide film, or an SOI (silicone on insulator) wafer.

Further, a method may also be adopted wherein a filter, a rib, and a well are individually formed by etching a silicon wafer by an etching method and they are stacked by taking advantage of the smoothness of the silicon wafer to prepare a chip. In this case, the thickness of the filter, the thickness of the rib, and the thickness of the silicon wafer for wells are each independently properly determined. Regarding the etching method, dry etching and wet etching each independently can be selected for each of the filter, the rib, and the well.

In this method, unlike the method in which the filter and the rib or the well are etched using an identical material, there is no need to provide any hole having a difference in level, and what is required is only that, after through-holes having diameters determined respectively for the filter, the rib and the well are formed, they are stacked, and, thus, the assembly can be simply prepared. Further, since joining is carried out by interface bonding, in which joining is carried out by taking advantage of smoothness of silicon without using any adhesive, a chip, which is free from liquid leakage from the interface and has high peel strength, can be prepared.

In order to stack the filter, the rib, and the well at predetermined positional spacings, preferably, registration marks are previously provided in them, and registration is carried out based on the mark. When stacking is carried out in a liquid, the lamination plane is preferably clamped to prevent the separation of the lamination plane by lateral slipping of the laminated plate.

In the third method, the filter is prepared by anodization of a metal. Object metals include aluminum and silicon. The third method will be specifically described by taking aluminum as an example. An aluminum plate is first provided. In the aluminum plate, preferably, a shallow concave face having a depth about 100 µm smaller than the depth of the well is if necessary previously formed on one side of the aluminum plate. For example, when the depth of the well is 5 mm and the thickness of the filter is 20 µm, an aluminum plate having a thickness of 5.0 mm and provided with a concave face of 4.9 mm is provided. From the viewpoint of maintaining mechanical strength during anodization, a method may also be adopted in which a filler such as wax such as montanoic acid wax or polyethylene wax (manufactured by Clariant K.K.) is filled into the interior of the concave face and, after filter formation, is removed by heat melting.

Subsequently, on the opposite side of the concave face, leading pores for filter pore formation are formed. Preferably, the leading pores have a pitch of not more than 1 µm, preferably not more than 0.5 µm, and a depth of not less than 0.1 µm. These leading pores may be formed by not only stamping using a mold, but also photoetching using a resist.

The aluminum is then anodized while applying voltage in an oxalic acid or sulfuric acid solution. A specific method is described in Japanese Patent Laid-Open No. 11099/2003. After the preparation of the filter by anodization, in order to extend the bottom face in the concave face formed in the aluminum to the filter pores, a strong acid such as phosphoric acid, hydrofluoric acid, or nitric acid, or a mixed acid composed of them, or ferric chloride is dropped on the concave face of the aluminum well. For example, in the case of ferric chloride, the well bottom face can be extended to the pore face of the filter by etching at 50°C for 20 hr. These methods are described, for example, in OYO BUTSURI, Vol. 69, No. 5 (2000) and Japanese Patent Laid-Open No. 258650/2000. According to the anodization method, a filter having a pore diameter of 5 to 450 nm, an aspect ratio of about 100, and a size of about of 5 to 10 mm can be prepared.

In the fourth method, the filter is prepared by nanoprinting of a resin. At the outset, a base plate for filter formation by nanoprinting is provided. The base plate may be either a smooth plate or a plate having a concave face in which an about 100 µm-thick bottom part is previously left for well hole. As with the above case, this concave face may be filled with wax or the like.

A mold for nanoprinting is previously provided. The production process of the mold is not particularly limited. However, regarding a mold provided with pores having a diameter of not more than several tens of micrometers, the production process thereof is limited. In general, the mold is prepared by the so-called MEMS (micro electro mechanical system) techniques such as anisotropic etching utilizing crystal anisotropy of silicon or X-ray lithography. Alternatively, a metal-plated reversed mold prepared based on the mold prepared by the MEMS technique may be used. The mold thus prepared is mounted on a pressing device. Nanoprinting devices include NPHT-1 prepared by Tectoria Co., Ltd., a nanoprinting device manufactured by Hitachi, Ltd., and a nanoprinting device manufactured by MEISHO KIKO K.K.

The resin for molding by nanoprinting is preferably a resin having good fluidity, and, for example, liquid crystal polymers and crystalline nylon resins are suitable. Alternatively, a method may also be adopted in which an oligomer such as an epoxy resin or urethane acrylate is photocured or heat cured simultaneously with pressing. The epoxy resin and the urethane acrylate may be in a liquid resin form or a prepreg film form.

When the filter which has been press molded by nanoprinting is not subjected to demolding and a skin-like resin is left on the bottom face, molding is less likely to be failed and demolding is easy. From the viewpoints of easiness on demolding and damage to the mold, the aspect ratio of the pores is preferably not more than 10, more preferably not more than 5.

Further, a method may also be adopted in which, at the time of molding, the skin part is intentionally left as the well part arid, separately, the well is formed by boring or etching of the skin part. When the film residue is utilized as the well, a method may be adopted in which, after the separation of the resin residue sheet from the base plate, the separated part is drill bored or photoetched to form through holes to the filter part. Alternatively, a method may also be adopted in which mechanical boring is carried out partway, and a final part of several tens to several hundred micrometers is then removed by etching with an acid, an alkali or the like.

In the fifth method, a rib or a well is prepared by photomolding. At the outset, a filter is prepared by any of the electroforming, etching, anodization of a metal, and nanoprinting of a resin. It is also possible to use, for example, a filter prepared by pressing or an expanded metal method. The above filter is used as a base plate or is fixed onto a different base plate, and a well or a rib is formed thereon.

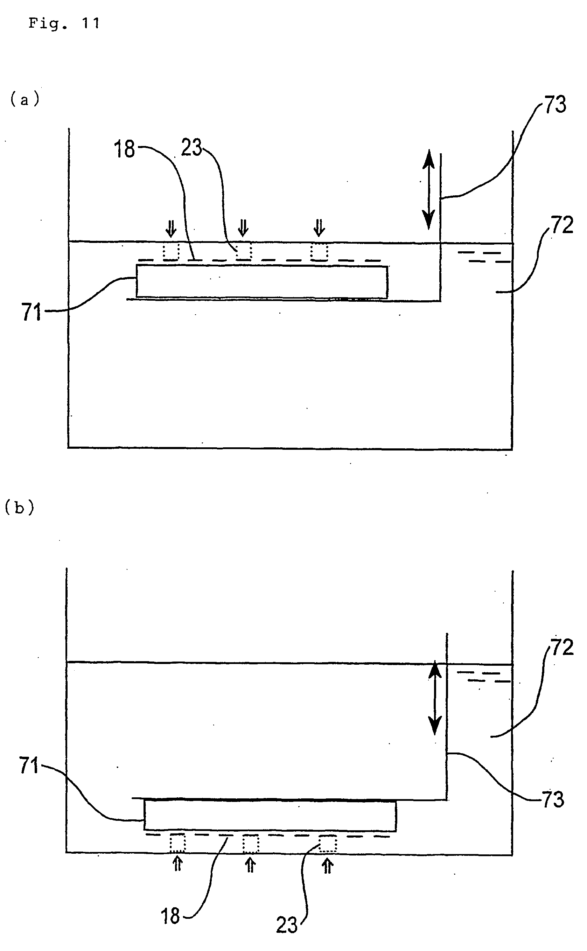

Specifically, as shown in Fig. 11 (a), a filter 18 is fixed on a base plate 71, and the assembly is installed in a photomolding machine bath 72. An elevator 73 for supporting the base plate 71 is lowered, a UV curable resin is cast into the a filter 18 side in an amount corresponding to one layer, followed by the application of UV light to cure a rib part 23 (or a well part) of a resin layer in an amount corresponding to one layer. The movement of the elevator 73 and UV irradiation are repeated until predetermined layers are stacked and cured. Thereafter, a laminate including the base plate 71 is taken out of the UV resin liquid, the uncured part is washed away, the base plate 71 is removed, followed by post curing to prepare a filter provided with a rib or a well.

In this method by photomolding, when a closed rib part 23 or well is formed by photomolding on the filter 18 disposed in a lower position, the discharge of the resin remaining uncured within the interior is difficult and the internal resin is raised by surface tension, possibly leading to disturbance of the rib or well shape. For this reason, as shown in Fig. 11 (b), a preferred method is to allow a rib part 23 or a well to be formed and grown by photomolding on the lower side of a filter 18. A photomolding apparatus for this method is commercially available from DENKEN CO., LTD. under the tradename SLP-4000, and the molding is entrusted. In the current photomolding, a photocurable resin is mainly cured by laser beam irradiation. In the above case, the irradiation pattern is uniform, and stacking of a UV resin by photoirradiation through a photomask is also possible. This can shorten molding time.

In the sixth method, the well or the rib is joined to the filter by heat bonding. For example, the filter part is heated, and the well or rib is joined in a nonheated state for heat bonding. The filter part is formed of a metal or ceramic having good thermal conductivity, and the rib is formed by molding using a thermoplastic material. The end face of the filter is joined to the heating material, and the filter is heated to heat bond the well and the rib by taking advantage of heat conductivity.

Alternatively, electromagnetic induction heating or dielectric heating is utilized. The well or rib used herein is formed of a thermoplastic resin that is a low-permittivity material which does not generate heat upon electromagnetic induction heating or dielectric heating. The permittivity of the material used herein is preferably not more than 3.5. Specific resin material include, for example, polyethylenes, polypropylenes, polystyrenes, polymethyl methacrylate resins, liquid crystal polymers, polycarbonates, polyamide resins, polyethylene terephthalates, polyethylene naphthalates, cycloolefins, polymethylpentenes, polyarylates, polysulfones, and polyethersulfones.

The resin material is previously molded by any method into a well or a rib. In this case, preferably, all of a plurality of wells mounted on the filter are integrally prepared, and methods usable herein include a method in which a continuous pipe having a plurality of well holes is formed by injection molding, press molding, or profile extrusion molding and the pipe is cut into rounds and a method in which holes are formed, for example, by drilling in a plate.

The filter material is preferably a material which is heated by dielectric heating and preferably has a permittivity of not less than 3.8. Specific examples of materials usable herein include: metals such as gold, silver, nickel, copper, iron, aluminum, titanium, silicon, stainless steel, tungsten, and molybdenum; metal oxides such as silica, alumina, and titania; metal nitrides such as SiN; and glass such as soda glass, borosilicate glass, Pyrex (trademark) glass, and quartz glass. Alternatively, a mixture of a resin material with a filler of an inorganic material may also be used.

Any one of the well and the rib is put on top of the filter, for example, by sandwiching a filter between the well and the rib, and the assembly is fixed by a fixture. Alternatively, interposition is carried out by a resin roller or the like, and electromagnetic induction heating or dielectric heating is carried out under pressure to generate, in the filter part, heat which is utilized to heat weld the well and the rib to the filter. The frequency of electromagnetic induction or dielectric heating may be 30 kHz to 300 MHz, preferably 1 to 100 MHz.

In the seventh method, the filter is bonded to the well with the aid of an adhesive. An adhesive is previously coated on the end face of one side of the well, and the coated well is applied to the filter, optionally followed by heating for bonding. Alternatively, a method may also be adopted in which wax such as LICOWAXLP manufactured by Clariant Japan is coated onto the well and the filter is heated followed by coating and cooling for bonding.

In the eighth method, a well or a rib is prepared by insert molding of a filter. In the insert molding, the previously prepared filter can be inserted into a mold, followed by resin molding for integral molding of the filter and the resin.

In the ninth method, a filter and a well are bonded to each other by magnetic force using a magnetic material. For example, a ferromagnetic body is used in any of the filter and the well, and the corresponding well or filter may be formed of a magnet, followed by connection of the filter to the well through the action of the magnet to prepare a well with a filter. Ferromagnetic bodies include nickel and iron, and magnets include ferrite, samarium-cobalt magnets, neodymium magnets, and aluminum-cobalt magnets.

When the filter is formed of a magnet, a method may be adopted in which gold is vapor deposited to a thickness of several tens of angstroms onto the surface of a smooth metal plate having good adhesion to gold, for example, copper, nickel, or chromium. Subsequently, after coating of a photoresist, patterning is carried out so that the resist stays in parts where pores of the filter are formed. Next, electroless ferrite plating is carried out to form a 1 to 20 µm-thick ferrite layer, the resist is then peeled with a resist peel liquid, and, finally, the ferrite layer is peeled off from the gold deposited layer. This ferrite plating may be carried out under conditions described, for example, in Transactions of the Magnetics Society of Japan, Vol. 22, No. 9, 1998 1225-1232 and Transactions of the Magnetics Society of Japan Vol. 24, No. 4-2, 2000, 515-517.

Thus, the filter is formed of a magnet, and, for example, the well is prepared by electroforming nickel plating, followed by connection of both materials through the action of the magnetic force to prepare a well with a filter.

When the rib is formed of a magnet, a well with a filter may be prepared, for example, by mixing a ferrite, samarium or neodymium magnetic powder with a resin binder, forming the mixture into a plastic magnet sheet or the like (for example, manufactured by MagX Co., Ltd.), forming holes having a thickness of about 0.3 mm to 1 mm by a laser beam, and then connecting the well to the nickel filter through the action of the magnet.

In the tenth method, the well is connected to the filter by mechanical fitting. For example, before the separation of the resist of the well prepared by electroplating of nickel, the resist is further coated and patterned to provide, on the center of the upper surface of the well wall, a tapered resist groove of which the width is 50 µm to 100 µm smaller than the width of the well. The groove is filled by plating with a soft metal such as tin or gold, or a resin. Subsequently, the resist is peeled off with a peeling liquid to form a well having a convex-type structure in which comb-shaped convex parts of tin, gold, a resin or the like having a height and a width identical to each other have been formed on the nickel well. Simultaneously, on the filter side as well, a concave shape of nickel, polyimide resin or the like, harder than the convex part of the well, which serves as a female die and has the same height and width as the convex form, is formed with the resist on the same position. Next, both the materials are connected to each other by pressing using a roll or the like for mechanical fitting to prepare a well with a filter. Further, a method may also be adopted in which, conversely, a concave shape is provided on the upper surface of the well wall and a convex shape is provided on the filter side.

In the biochip according to the present invention, the fist filter may be provided at the bottom part of the well and, in addition, a second filter may be provided on the upper side (provided opposite to the first filter through the well). The second filter may be provided by placing probe-supported particles within the well having a filter at its bottom part formed by the above method and then joining the filter formed by the above method to the well, for example, by an adhesive, magnetic force, mechanical fitting, planar junction between smooth surfaces, or mechanical clamping. Thus, a biochip provided with upper and lower filters can be prepared.

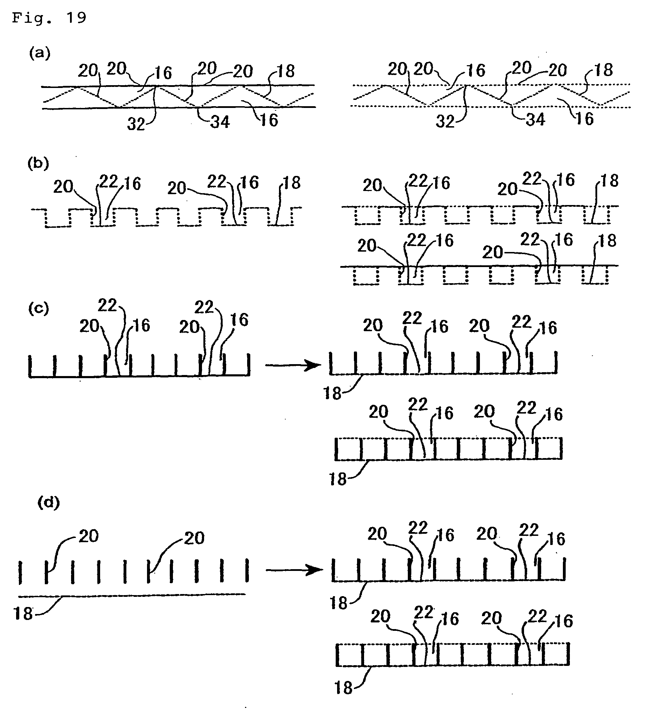

The biochip having a filter at the bottom part of each well may be prepared, for example, by the following methods:
(1) a method in which, as shown in Fig. 19 (a), a filter 18 in a corrugated form is provided and the convex apex part 32 is bonded to the concave apex part 34 through a suitable film or filter constituting a side wall 20 to form each well 16, 16 ···;
(2) a method in which, as shown in Fig. 19 (b), a metal or resin filter is molded by a press mold to form concaves, at predetermined spacings, as wells 16, 16 ···, or a method in which, on one side thereof, another filter or film is joined, for example, by an adhesive, a magnet, or mechanical flitting to form wells having a closed space;
(3) a method in which, as shown in Fig. 19 (c), through-holes are formed on the bottom wall 22 in the wells 16, 16 ···, for example, by laser, press, or photoetching to form a filter 18, or a method in which, further, the top of the wells is joined to a filter by a pressure-sensitive adhesive, a magnet, or mechanical fitting;
(4) a method in which, as shown in Fig. 19 (d), wells, which are free from the bottom wall 22 and constituted by side walls 20, are prepared and a filter 18 is bonded to the bottom part of the wells, or a method in which, further, a filter is joined onto the wells by a pressure-sensitive adhesive, a magnet, or mechanical fitting to cover the wells; and
(5) a method in which a metal, a metal oxide, a metal/metal oxide, a resin or the like is bored by a laser beam, mechanically pressed or photoetched.

The biochip according to the present invention has a plurality of integrally formed wells or a single well, and a dispersion with a probe-supported particle dispersed therein is contained in the well. The particle contained in the well has a diameter larger than the filter pore and, when the particle in the chip is allowed to stand on the filter pore for optical detection, the diameter of the probe-supported particle and the diameter of the filter pore satisfy a relationship represented by formulae: particle diameter/pore diameter = 1.1 to 2.5 and particle diameter < pore spacing < particle diameter x 10, more preferably particle diameter < pore spacing < particle diameter x 4. Here the term "pore spacing" refers to the shortest distance of a pore-free part between adjacent pores.

When particle diameter/pore diameter is less than 1.1, the particle enters the pore, leading to an increase in probability that the particle cannot be detached from the pore. When particle diameter/pore diameter exceeds 2.5, in filtering a particle-containing solution through a filter to allow the particle to stay on the filter, there is an increasing tendency that the particle is not located on the pore and particles are arranged on the filter either randomly or in such a state that particles are put on top of each other. In order to further prevent particles from entering pores and disabling the detachment of the particles from the pores, the particle diameter/pore diameter is more preferably 1.15 to 2.0, still more preferably 1.2 to 1.6.

When the chip is used for isolation and purification, the particle diameter of the particle, the pore diameter of the filter, and the pore spacing of the filter are set so that they satisfy a relationship represented by formula: particle diameter > pore diameter + pore spacing/2. When this relationship is satisfied, in filtering the particle-containing solution through the filter to allow the particles to stand on the filter, the particles do not stand on all the filter pores but instead stand at such a pore spacing that at least one pore, on which no particle stand, is present between pores on which the particle stands. This means that filter pores not clogged by the particle are present at least 50% spacing, and, as a result, an increase in filtration resistance is prevented.

The total number of particles contained in one well varies depending upon applications and, for use in isolation and fractionation, the total number of particles is 100 times to 1/100 time, preferably 10 times to 1/10 time the number of filter pores, while, for use in detection and identification, for each probe, the absolute number of particles is 1 to 1000, more preferably 1 to 500, still more preferably 1 to 200. The larger the number of particles per well, the larger the filtration resistance of the filtration and the lower the filter performance or the higher the fear of causing destruction of the filter. Even 1000 particles suffice as the number of particles useful for the detection and identification, and the number of particles which exceeds 1000 particles is insignificant and rather makes it difficult to perform filtration.

The absolute number of probe-supported particles contained in each well can be regulated by the following method.

Specifically, when a particle solution is introduced, the regulation can be carried out by introducing particles while actually counting individual particles by means of a CCD camera or the like and while individually regulating the particles by means of a flow cytometer. Alternatively, a method may also be adopted in which the particle concentration of the particle solution is previously measured, the number of particles is calculated based on the particle concentration of the solution and the specific gravity of the particle, the amount of the particle solution calculated back from the results is introduced by a spotter or the like, whereby the approximate number of particles can be introduced into the cell chamber. A method may also be adopted in which a predetermined number of particles are spotted a plurality of times, the number of spotted particles is counted each case to calculate the amount of particle which is insufficient, and the spotting is repeated until the amount which is insufficient is close to zero.

A certain level of identity suffices for the number of particles, and the error range is preferably not more than 20%, still more preferably not more than 10%, in terms of CV value.

The particle on which the probe is supported is not particularly limited so far as the diameter of the particle is larger than the diameter of the pore in the filter, and any of organic particles, inorganic particles, organic inorganic particles, phase change gel and the like may be used.

Specifically, regarding organic particles, particles prepared by emulsion polymerization or suspension polymerization of a monomer raw material comprising a single or two or more monomers selected from butadiene, styrene, divinylbenzene, acrylonitrile, acrylate, methacrylate, acrylamide, benzoguanamine, nylon, polyvinyl alcohol, fluoro and other monomers may be used. Alternatively, a method utilizing membrane emulsification or the like may also be adopted in which a resin is extruded through a porous plate with uniform pores to prepare particles. These particles may be if necessary classified for collecting uniform particle diameters by a classifier.

Further, a product prepared by adding a magnetic body such as ferrite during or after polymerization, or a product prepared by plating particles with ferrite by a method such as Japanese Patent Laid-Open No. 83902/1998, or a naturally occurring product crosslinked gel of cellulose, starch, agarose, galactose or the like, or a synthetic crosslinked gel of acrylamide or the like may also be used.

The particle diameter distribution of particle diameters is preferably not more than 10%, more preferably not more than 5%, in terms of CV value. When the CV value exceeds 10%, the probability of the entry of particles into pores in the filter is enhanced.

Styrenic particles may be prepared by a method described, for example, in Japanese Patent Laid-Open No. 168163/1982.

Magnetic particles may be prepared by a method described, for example, in Japanese Patent Laid-Open No. 176622/1999.

Inorganic particles usable herein include metal oxide particles, metal sulfide particles, and metal particles.

Silica particles are the most preferred metal oxide particles, and various commercially available silica particles may be used.

Further commercially available particles usable herein include, for example, IMMUTEX (tradename: JSR corporation), MCI-GEL (tradename: Mitsubishi chemical corporation), Toyopearl (tradename: TOSOH Corporation), DAISOGEL (tradename: DAISO., LTD), Shodex (tradename: Showa Denko K.K.), Sunsphere (tradename: Asahi Glass Co., LTD), PL-PEGA (tradename: Polymer Laboratories), PL-CMS (tradename: Polymer Laboratories), PL-PBS (tradename: Polymer Laboratories), PL-DMA (tradename: Polymer Laboratories), AM resin (tradename: Novabiochem), P500 (tradename: Toray Industries, Inc.), Toraypearl (tradename: Toray Industries, Inc.), Techpolymer (tradename: Sekisui Plastic Co., Ltd.), MP series and MR series (tradename: Soken Chemical Engineering Co., Ltd.), BELLPEARL (tradename: Kanebo. Ltd.), EPOSTER (tradename: Nippon Shokubai Kagaku Kogyo Co., Ltd.), cellulose powder (Chisso Corp., Asahi Chemical Industry Co., Ltd.), Sephacell (tradename: Amarsham-Pharmacia), and Sephallose (tradename: Amarsham-Pharmacia).

The surface of the above particles may be modified by, for example, by various functional groups, such as amino, carboxyl, carbodiimide, epoxy, tosyl, N-succinimide, maleimide, thiol, sulfide, hydroxyl, trimethoxysilyl, nitriletriacetic acid, benzosulfoamide, polyethyleneimine, quaternary ammonium, and octadecyl groups, or γ-glycidoxypropyltrimethoxysilane to form a probe-binding site.

In the surface modification, in order to lower steric hindrance in a reaction of the particle-supported probe with an analyte, compounds having a structure in which a functional group is attached to both ends of an alkylene group having 10 to 100 carbon atoms, for example, ethylene glycol diglycidyl ether derivatives, N-k-maleimide undecanic acid, mercaptopropyltrimethoxysilane, calixarene derivatives, and liquid crystals, are usable as a spacer. Oligonucleotides modified, for example, by an amino, carboxyl, or thiol group are usable as a site which serves both as a spacer and a spacer which serves also as a binding site.

When organic particles are used as the particle on which the probe is to be supported, the particle diameter is preferably 0.02 µm to 120 µm, more preferably 0.1 µm to 60 µm. When the particle diameter is below the lower limit of the above-defined range, handleability is poor and, consequently, the preparation of a filter for capturing these particles becomes difficult. Further, in this case, the filter pores are so small that the analyte is likely to clog the pores. When the particle diameter is above the upper limit of the above-defined range, the reactivity is sometimes lowered due to steric hindrance.

If necessary, the particles are preferably in a porous or hollow form. In this case, even when the particle diameter is large, a lowering in reactivity with the analyte caused by settling of particles by the weight can be prevented.

When inorganic particles are used as the particle on which the probe is to be supported, the particle diameter is preferably 0.1 µm to 0.1 mm, more preferably 1 µm to 0.05 mm. When the particle diameter is below the lower limit of the above-defined range, handleability is poor and, in particular, capturing particles by the filter pores becomes difficult. On the other hand, when the particle diameter is above the upper limit of the above-defined range, settling occurs, often leading to a lowering in reactivity.

If necessary, the particles are preferably porous. In this case, even when the particle diameter is large, for example, settling of particles by the weight can be prevented.

The probe-supported particle may be contained in each well by previously separately immobilizing various probes corresponding to respective wells onto particles and introducing the probe-supported particles into corresponding cells by a spotter or the like. Alternatively, a method may also be adopted in which particles having a probe-binding site on the surface thereof are introduced into individual wells by a spotter or the like and probes different from each other in type and corresponding to respective predetermined wells are introduced into corresponding wells by a spotter or the like.

In the former method, solid phase synthesizers commercially available from various companies may be used for binding a probe to the particle, and examples of solid phase synthesizers include oligonucleotide synthesizers, peptide synthesizers, sugar chain synthesizers, and various low molecular compound synthesizers manufactured by Combinatorial Chemistry. In the solid phase method, silica beads or polystyrene divinylbenzene particles are used as carriers for the reaction and separation. When the solid phase synthetic carrier beads are used also as particulate carriers contained in the chip 10, the probe-support beads synthesized by the synthesizer as such may be contained in the chip 10 by spotting. These synthesizers, particularly oligonucleotide synthesizers, are generally spread. At the present time, for example, when a synthesizer is set in the evening, particles with an oligonucleotide immobilized thereon can be provided on the following morning. An order made chip containing any desired nucleotide can be prepared in a very short time simply by spotting this oligonucleotide-supported particle.

Probes supportable on the particle include, for example, nucleic acids, proteins having a molecular weight of 500 to 1,000,000, lipids, sugar chains, cells, protein expression cells, aptamers, viruses, enzymes, pharmacologically active lead compounds having a molecular weight of 50 to 1,000,000, or chemical compounds which have specific physiologically activity or have a possibility of having physiologically activity.

Among them, proteins having a molecular weight of 50 to 1,000,000 include specifically synthetic peptides, membrane proteins, enzymes, transport proteins, cytokines, lymphokines, antibodies such as IgA and IgE, various antigens, or proteins having bioluminescent function such as luciferins, luciferases, aequorins, and green fluorescent proteins.

Specific examples of lipids include phosphatidic acid, phosphatidylinositol, mannoside, urushiol, and various gangliosides.

Aptamers are proteins, enzymes, dyes, amino acids, nucleotides, growth factors, gene expression regulators, cell adhesion molecules, and functional nucleic acids having a capacity of binding to bions or the like, and specific examples thereof include thrombin aptamers, elastase aptamers, activated putein C, and NS3 protease aptamers of hepatitis C virus.

Specific examples of low molecular lead compounds having a molecular weight of 50 to 1,000,000 include substrates, coenzymes, regulators, lectins, hormones, neurotransmitters, antisense oligonucleotides, ribozymes, aptamers or other ligands, phenylpiperidine derivatives, sulfonamide/sulfonic acid derivatives, steroids, prostaglandin derivatives or other drug candidate compounds.

The probe-supported particle can have at least one identification means for providing probe identification information for identifying the type of the probe. Such identification means include colors, shapes and diameters of probe-supported particles.

A plurality of these identification means may be used in combination. For example, a combination of color and particle diameter, a combination of color and shape, and a combination of color and gene sequence are possible. When the color is used as the identification means, the particle is impregnated and colored with a dye, a pigment, a fluorescent dye or the like. Regarding dyes, pigments, fluorescent substances, and phosphors, a combination of colors having different absorption and luminescence wavelength with color concentrations can provide a larger number of probe identification information. For example, when the number of types of color is 2 and the number of color concentrations is 3, 9 types of identification information can be provided. For example, IMMUTEX manufactured by JSR Corporation may be used as dye particles. Fluorescent particles include, for example, carboxyl-modified, sulfonic acid-modified, aldehyde-sulfonic acid-modified, and amine-modified particles that are sold as fluospheres fluorescent microspheres by Molecular Probes, Inc.

These color identification information may be detected as follows. At the outset, color-labelled particles are exposed to a light source. When the coloring matter label is a fluorescent substance or a phosphor, the light source should be a light source with corresponding fluorescence or phosphorescence excitation wavelengths. Next, for example, fluorescence or phosphoresce emitted from particles is optically detected, for example, with a CCD camera or a photomultiplier tube, and the detected information is statistically processed to obtain probe identification information.

When the particle diameter is used as the identification information, particles having diameters previously varied according to respective probes are provided. Regarding the particle diameters, the particle diameter difference is preferably not less than 0.3 µm unit, more preferably not less than 0.5 µm, from the viewpoint of detection sensitivity.

Thus, when a plurality of types of identifiable probe-supported particles are contained in a well, simultaneous multi-item detection can be realized in one well. This can shorten the reaction time and the operation process. Further, when a chip having a plurality of wells is provided and information about well position is combined with a plurality of identifiable probe-supported particles, for example, if the number of wells is 100 and the number of types of identifiable probe-supported particles is 100, a biochip which can contain 10,000 (100 x 100 = 10,000) types of probes can be realized.

The probe-supported particles having the above-described identification means can be contained in each well as follows.
(i) A plurality of probe-supported particles which are identical to each other in probe identification information in all of said identification means are contained in an identical well and wells are identical to each other in said probe identification information for a plurality of probe-supported particles contained therein. That is, a plurality of particles, which are identical to each other in probe identification means such as color or particle diameter, as well as in identification information such as the type of color and the size of the particle diameter, are contained in one well. Further, the wells are identical to each other in type of particle contained therein. In this connection, it should be noted that, when the fact that identification information is identical is previously known, the probe identification information and the probe identification step can be omitted.
(ii) A plurality of probe-supported particles which are identical to each other in probe identification information in all of said identification means are contained in an identical well and wells are different from each other in said probe identification information for a plurality of probe-supported particles contained therein. That is, a plurality of particles, which are identical to each other in probe identification means such as color or particle diameter, as well as in identification information such as the type of color and the size of the particle diameter, are contained in one well. Further, the wells are identical to each other in identification means but are different from each other in identification information such as the type of color and the size of particle diameter of the particle contained therein.
(iii) A plurality of probe-supported particles which are different from each other in probe identification information in at least one identification means are contained in an identical well and, regarding the plurality of probe-supported particles contained in the wells, the wells are identical to each other in the construction of said identification information in all the identification means. That is, a plurality of probe-supported particles which are different from each other in identification information such as the type of color and the size of the particle diameter for at least one of probe identification means such as color and particle diameter are contained in one well, and the wells are identical to each other in the construction of the identification information.
(iv) A plurality of probe-supported particles which are different from each other in probe identification information in at least one identification means are contained in an identical well and, regarding the plurality of probe-supported particles contained in the wells, wells are different from each other in the construction of the identification information in the at least one identification means. That is, a plurality of probe-supported particles which are different from each other in identification information such as the type of color and the size of the particle diameter for at least one of probe identification means such as color and particle diameter are contained in one well, and the wells are different from each other in the construction of the identification information.

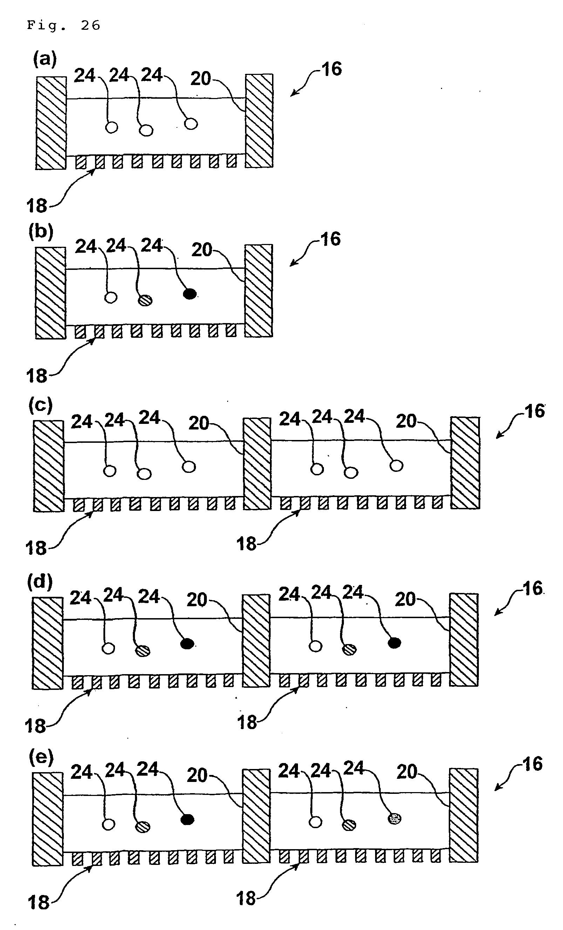

According to the contents of the assay, the number of wells and the construction of probe-supported particles contained in the wells are properly selected. Embodiments regarding this include: an embodiment as shown in Fig. 26 (a) in which identical probe-supported particles are contained in a single well; an embodiment as shown in Fig. 26 (b) in which probe-supported particles different from each other in identification information are contained in a single well; an embodiment as shown in Fig. 26 (c) in which identical probe-supported particles are contained in a plurality of wells; an embodiment as shown in Fig. 26 (d) in which probe-supported particles different from each other in identification information are contained in a plurality of wells and the wells are identical to each other in the construction of the identification information; an embodiment as shown in Fig. 26 (e) in which probe-supported particles different from each other in identification information are contained in a plurality of wells and the wells are different from each other in the construction of the identification information.

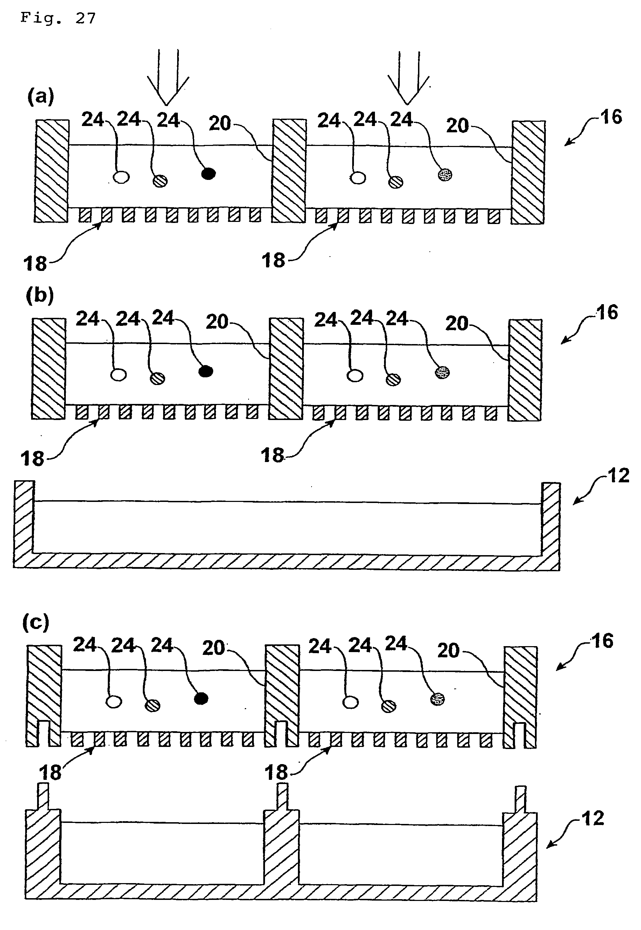

For example, in the introduction of an analyte in the wells containing probe-supported particles, as shown in Fig. 27 (a), the analyte can be introduced through the upper opening in the well 16. Alternatively, as shown in Fig. 27 (b), one analyte contained in a container 12 may be brought into contact with the bottom part of each well 16. Further, as shown in Fig. 27 (c), different analytes can be contained in respective wells 16. Specifically, a method may also be adopted in which a vessel 12 having compartments corresponding to respective wells 16 is provided, different analytes are contained in respective compartments, and the bottom parts of the wells 16 are brought into contact with the respective analytes.

### <Biochip kit>

The biochip kit according to the present invention includes a biochip and a vessel for containing a well(s) in the biochip or for connection to the biochip.

The material for the vessel is not particularly limited, and any of organic materials and inorganic materials may be used. Specific examples of organic materials include polyethylenes, polyethylene vinyl acetate resins, polyethylene vinyl resins, polypropylenes, polystyrenes, polybutadienes, polyacrylonitrile resins, polymethyl methacrylate resins, AS resins (copolymer of acrylonitrile with styrene), ABS resins (copolymer of acrylonitrile with butadiene and styrene), AAS resins (copolymer of acrylonitrile with acrylic ester and styrene), polycarbonates, polyamide resins, polyethylene terephthalates, polyethylene naphthalates, aliphatic polyesters, polylactic acids, polyglycolic acids, aliphatic polyamides, alicyclic polyamides, cycloolefins, polymethyl pentenes, polyvinyl chlorides, polyvinyl acetates, polyarylates, polysulfones, polyethersulfones, polyimides, triacetylcelluloses, cellulose acetate resins, cellulose nitrate resins, epoxy resins, polytetrafluoroethylenes, fluorinated ethylene polypropylene copolymers, tetrafluoroethylene perfluoroalkoxy vinyl ether copolymers, polychlorotrifluoroethylenes, ethylene tetrafluoroethylene copolymers, poly(vinylidene fluorides), poly(vinyl fluorides), and silicone resins.

These organic materials may be molded, for example, by injection molding, pressless molding, injection compression molding, injection press molding, compression molding, transfer molding, cutting, or photomolding.

Specific examples of inorganic materials include: metals such as nickel, copper, iron, aluminum, titanium, and silicon; metal oxides such as silica, alumina, and titania; and glass such as soda glass, borosilicate glass, Pyrex (trademark) glass, and quartz glass.

These inorganic materials may be formed into a vessel shape by various methods, for example, molding or surface treatment methods, typified by press molding, plate etching, laser boring, sandblasting, and surface coating of resin or metal vessels.

Further, a method may also be adopted in which a plate having a hole(s) or a hole-free plate is used, and a plurality of these plates (one of or both the above types) are stacked on top of each other to form a predetermined vessel. In order to prevent liquid leakage from the stacked part, the plates in their surfaces which are to be stacked on top of each other should be smooth. To this end, the plates are polished for smoothening, or alternatively already smooth plates such as silicon wafers may be used. Joining between smooth plates can provide a very strong joint without use of any adhesive or the like.

When a silicon wafer is used as the plate, if necessary an oxide film may have been formed on the silicon wafer, or alternatively oxidation may be carried out after plate lamination.

The vessel using the above plate is prepared, for example, as follows. At the outset, a plate for the bottom of the vessel is first provided. This plate has minute air introduction/discharge holes. When deep air introduction/discharge holes are desired, if necessary, a plurality of plates are stacked as the lower most layer on top of each other. Any desired number of smooth plates having a shape identical to a shape formed by horizontally cutting the vessel are stacked thereon so that the hole of the vessel has a predetermined depth. The depth of the hole in the vessel can be properly regulated by stacking a plurality of plates having the same shape. When the regulation of the shape of the vessel in the direction of the depth is desired, for example, when a hole shape having a difference in level is desired, any desired hole shape can be provided by stacking plates having different shapes.

The lid of the vessel disposed on the uppermost part may be a plate having a vertically formed hole for liquid or air pressure introduction/discharge purposes. Alternatively, for example, a plate having a groove on its surface may be put as a lid on the uppermost part of the plate laminate. Further, the plate located at the uppermost part or bottom part of the laminate may be a transparent material such as glass so that optical detection is possible.

In general, the preparation of a hole having a very small diameter and a large depth, that is, the so-called high aspect ratio, is difficult. According to the above method, however, a vessel having a hole with any desired aspect ratio can be prepared.

Further, according to the above method, holes corresponding to wells in the chip may be provided in the vessel, or alternatively one hole common to a plurality of wells may be provided in the vessel.

When a vessel with wells corresponding to the wells in the chip is desired, plates having a section with hole shape similar to a horizontal section of the wells are stacked to the depth of the hole of the vessel.

When a vessel with one hole for a plurality of wells is desired, plates with a hole having a larger diameter than the area covering the horizontal sectional area defined by the plurality of wells are stacked to the depth of the hole of the vessel.

As described above, a vessel having a hole at its bottom part may be prepared by using, as the vessel bottom part, a plate with a hole previously formed therein. The provision of a hole at the bottom part of the vessel is advantageous in that, for example, after the well in the biochip is connected to the vessel, the liquid contained in the well in the biochip can be transferred to the vessel by applying pressure to the well in the biochip to discharge air through the hole provided at the bottom of the vessel.

The diameter of the hole at the bottom part of the vessel should be not more than a certain size because, when the hole diameter is large, the liquid in the vessel is leaked. The hole diameter is determined by the depth of the hole or the affinity of the hole for the liquid. In general, however, the hole diameter is 50 µm to 1 mm, preferably 0.1 mm to 0.5 mm. When the hole diameter is not more than 50 µm, clogging and increased filtration resistance occur. On the other hand, when the hole diameter exceeds 1 mm, there is a fear of causing liquid leakage.

The biochip and the vessel can constitute a biochip kit in which wells in the biochip are connected to vessel wells that correspond to the respective wells in the biochip and have the same diameter as the wells in the biochip. In this case, when the sectional area of the well on the downstream side of the transfer of the liquid is larger than the sectional area of the well on the upstream side of the transfer of the liquid, a registration error of the well at the connection part between the wells can be minimized.

The biochip and the vessel may be joined to each other by joining concave/convex, convex/concave or supersmooth faces at the connection part. The adoption of any one of the above joining methods can prevent the liquid from leaking from the joint to the adjoining well.

Further, in the same manner as described above, a plurality of biochips may be joined to each other by joining concave/convex, convex/concave or supersmooth faces at the connection part.

The connection between the biochip and the vessel or the connection between biochips can realize liquid transfer between mutual wells, that is, direct flow-down transfer of the solution between wells. In this case, since any transfer means such as a syringe is not required, unfavorable phenomena such as a reduction in amount of the solution due to the deposition of the solution onto the syringe wall and contamination do not occur. Further, the time necessary for solution transfer can be shortened.

The biochip kit comprising a biochip connected to a vessel can be prepared, for example, by the following method. In the method exemplified here, the vessel is formed by stacking a plurality of plates with a through-hole or free from any hole.

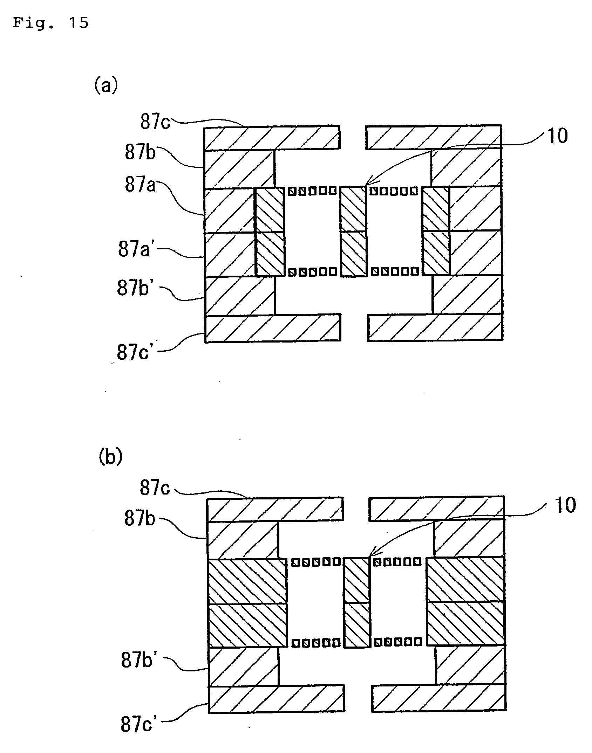

A biochip kit shown in Fig. 15 (a) is prepared as follows. At the outset, a plate 87a, which has a through-hole formed by etching a silicon wafer and has a thickness equal to the thickness of a biochip 10, and a plate 87b having any desired thickness, and a plate 87c having a gas or liquid introduction/discharge port or a liquid introduction/discharge groove formed by etching of a silicon wafer are provided. Next, while conducting registration, a plate 87b' is stacked onto a plate 87c', and a plate 87a' is stacked onto the plate 87b' to prepare a laminated plate having a hole with two level difference. One of chips in a biochip 10 before lamination is inserted into the hole of the plate 87a' in the laminated plate to prepare one half part biochip kit comprising the biochip composited with the vessel. Likewise, the plates 87a, 87b, 87c are stacked to prepare a laminated plate, and, further, the other chip in the biochip 10 before lamination is inserted into the hole to prepare another one half part biochip kit of the same.

Next, probe-bound particles are contained in the wells in the one half part kit through an open face in the well by spotting or the like. The one half part kit with the particles contained therein is used as a lower one half part kit, and the other one half part kit is used as an upper one half part kit. The upper one half part kit is put on top of and joined to the lower one half part kit so that the wells in the upper one half part kit face the wells in the lower one half part kit, whereby a biochip kit comprising a vessel provided integrally with a chip can be prepared. In this case, the registration can be accurately carried out by conducting registration between the upper and lower kits using previously provided registration marks.

Further, the construction of the biochip kit may be as shown in Fig. 15 (b). Specifically, a chip in which the outermost side upper and lower end faces are wide is provided. The upper and lower end faces of the biochip 10 may be applied and laminated onto one end face of a plate 87b (87b') having a through-hole formed by etching of a silicon wafer.

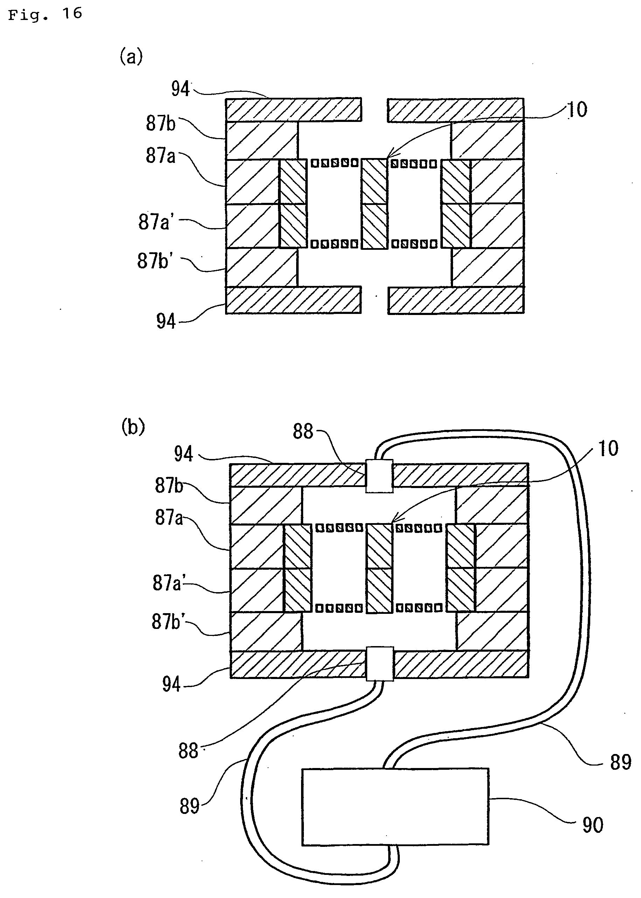

As shown in Fig. 16 (a), a smooth and transparent plate 94 formed of, for example, quartz glass may be used for optical detection as a plate that serves as the bottom part or lid part of the vessel. The optical detection is carried out for observing a reaction of particles filled into the filter. The construction as shown in the drawing can minimize the distance between the bottom part or the lid part and the filter and thus can improve the numerical aperture of an optical system. In this case, however, the volume of the vessel in which the liquid is contained is reduced, and, thus, in some cases, the amount of the analyte and the amount of the washing liquid which can be contained in the vessel are insufficient. This drawback of the insufficient volume of the vessel can be overcome by adopting a construction as shown in Fig. 16 (b). Specifically, a tube 89 connected to a pump 90 is mounted on the liquid introduction/discharge port 88 to circulate the liquid through the tube 89, or alternatively the liquid is moved vertically within the kit and only in a certain part of the upper and lower tube mounting ports to use a part of the tube as a kit vessel buffer.

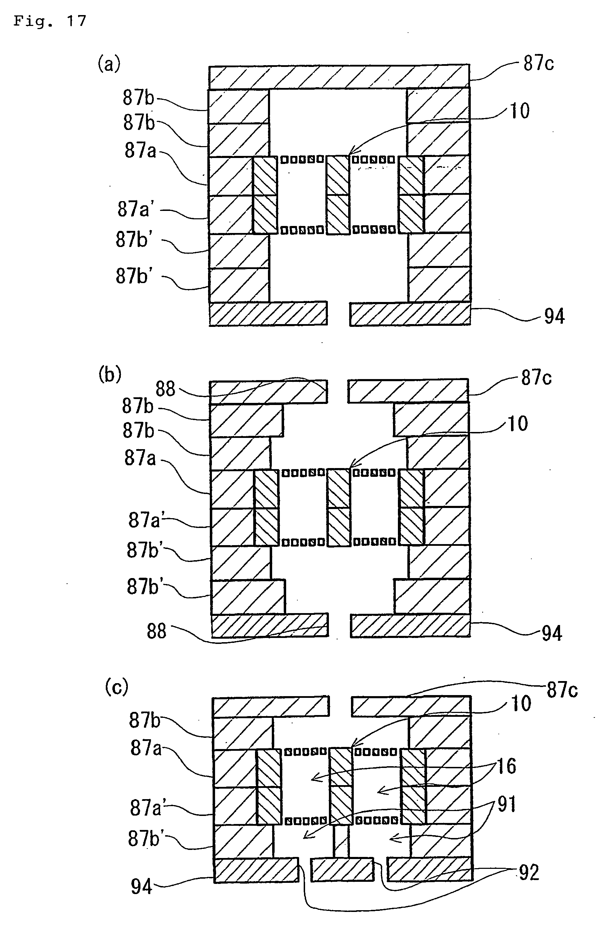

As shown in Fig. 17 (a), the volume of the vessel can be increased by stacking a plurality of plates 87b (87b'). Further, as shown in Fig. 17 (b), a slope can be given to the internal surface of the vessel by gradually varying the hole diameter of the plates 87b (87b'). In this case, the solution can be evenly introduced into or discharged from the wells in the biochip by giving a stepwise slope to the internal surface of the vessel in such a manner that the diameter of the vessel is gradually increased from the introduction/discharge port 88 toward the inward of the vessel.

As shown in Fig. 17 (c), a construction may also be adopted in which a vessel having independent wells 91, corresponding to wells 16 in the biochip 10, and holes 92 corresponding to these wells formed at the bottom part is connected. The diameter of the holes 92 at the bottom part of the vessel is preferably 100 to 500 µm, more preferably 150 to 300 µm, although the diameter also depends upon the height of the hole. When the hole diameter exceeds 500 µm, there is a fear of flowout of the liquid from the bottom of the vessel. On the other hand, when the hole diameter is less than 100 µm, pressurization or evacuation resistance is increased.

In the above biochip kit in which wells are provided in the vessel, after B/F separation of a target contained in an analyte by a probe bound to particles, the target is isolated from the particle by any method, and differential pressure is then applied to the upper vessel and the lower vessel, whereby the target contained in the well 16 in the biochip 10 can be transferred to the well 91 in the lower vessel. In order to evenly apply the differential pressure to each of the wells, any one of the upper vessel and the lower vessel is preferably a vessel having a housing space common to the wells in the biochip.

As described above, the volume of the vessel can be regulated as desired by stacking any desired number of plates having a hole. Further, a construction may also be adopted in which a transparent plate is used as at least one of the bottom part of the vessel and the lid part and, at the time of the detection, the transparent plate is located as the lower part to fill the filter pores with particles, and optical detection is conducted through the transparent plate while the target liquid trapped in the particles is separated and transferred to the wells in the vessel corresponding to the wells in the biochip.

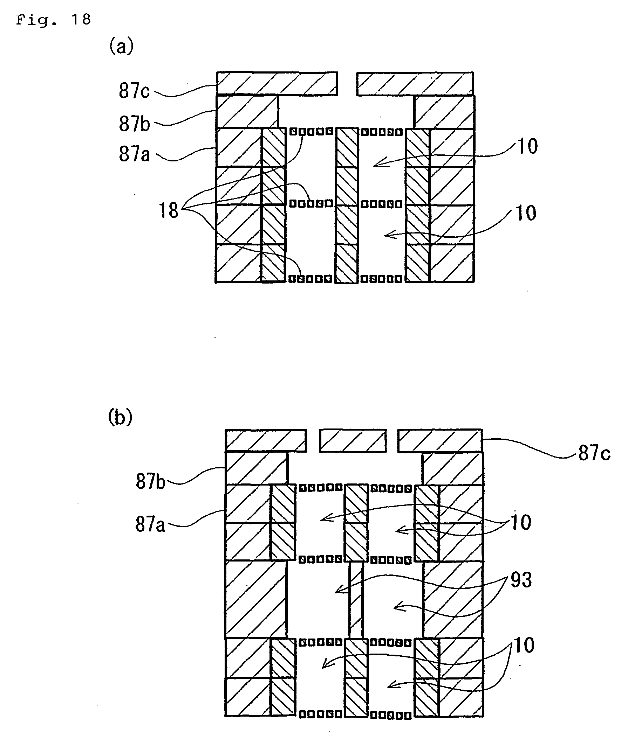

As shown in Fig. 18 (a), a construction may also be adopted in which biochips 10 are connected to each other so that the wells in one of the biochips 10 correspond to the wells in the other biochip 10. Further, as shown in Fig. 18 (b), a vessel having holes 93 corresponding to these wells may be provided between the biochips 10.

When particles having an identical probe are contained in each well in biochips disposed in multistage within the vessel, B/F separation capability can be improved by B/F separation using the probe-supported particles contained in the multistage chip. In the multistage chip, when the probe supported on particles introduced into the first-stage chip is different from the probe supported on particles introduced into the second-stage chip, for example, the so-called a tool for two step in vitro selection may be used.

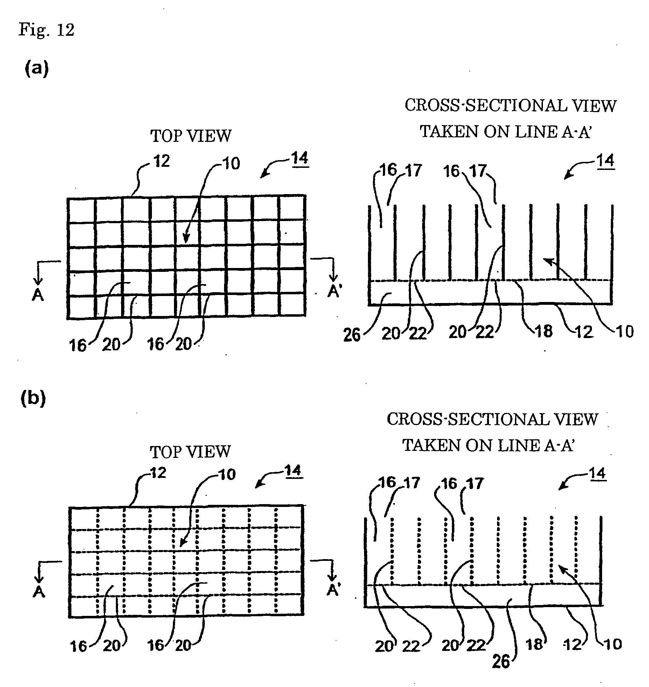

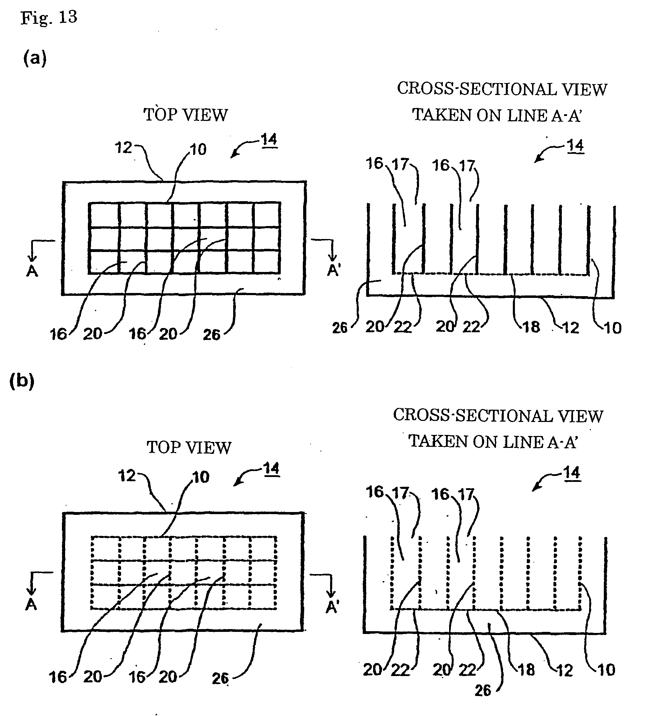

Figs. 12 and 13 each are a top view and a cross-sectional view taken on line A-A' showing one embodiment of the biochip kit according to the present invention. As shown in Figs. 12 and 13, a biochip kit 14 comprises a vessel 12 and a biochip 10.

The biochip 10 comprises a plurality of wells 16, 16 ··· partitioned by side walls 20. In these wells 16, 16 ···, for example, probe-supported particles are contained so that the wells 16 are different from each other in the type of the probe supported on the particle.

A bottom wall 22 in the well 16 is formed of a filter 18. This filter 18 cuts off the probe-supported particles contained in the well 16 and allows passage of various solutions containing an analyte, which is, for example, to be separated and detected through the biochip 10, various buffer solutions, washing solution, separating media, labelling agents, secondary antibodies, sensitizers, proteases, ionizing agents and the like.

Various solutions introduced into the vessel 12 can be introduced into or can be discharged from all the cells through a filter 18. Specifically, these various solutions can be introduced into any well 16 from the filter 18 or can be discharged from the well 16 through the filter 18 and a reservoir chamber 26, for these various solutions, which is adjacent to the well 16 through the filter 18, constituted by a space defined by the chip 10 and the vessel 12.

Alternatively, the side wall 20 may also be formed of the filter 18. In this case, transfer of various solutions between adjacent wells 16, 16 is also possible through the filter 18 formed in the side wall. In this case, the filter constituting the bottom wall 22 and the filter constituting the side wall 20 may be formed of the same type of material and contiguous to each other, or alternatively a composite filter in which the filter constituting the bottom wall 22 and the filter constituting the side wall 20 are different from each other in type.

As shown in Figs. 12 (a) and 12 (b), the vessel 12 and the chip 10 may be integrally formed of an identical material, or alternatively the vessel 12 and the chip 10 may be integrated by bonding and fixing the chip 10 to the vessel 12 within the vessel 12.

Alternatively, as shown in Figs. 13 (a) and 13 (b), the biochip kit 14 may be constructed from two elements, a vessel 12 and a chip 10 which are provided independently of each other, so that the chip 10 is housed within the vessel 12. In this case, for each operation, there is no need to always use the same vessel 12, and the vessel and the vessel in which the solution is contained may be changed according to the unit operation.

In the biochip kit 14 having the above construction, regarding probe-supported particles contained in the wells 16, the construction of the probe is specified according to the location of the well, and a plurality of probes can be contained on the chip without contamination.

A probe-supported particle solution contained in the biochip and a solution containing an analyte, various buffer solutions, washing solutions, separating media, labelling agents, secondary antibodies, sensitizers, proteases, ionizing agents or the like can freely go in and out between the wells 16, 16 ··· containing probe-supported particles. The above solution can be circulated into all the wells 16, 16 ··· by properly selecting the structure of the chip 10 or stirring conditions for the solution, whereby an opportunity to allow the solution to come into contact or to react with a plurality of types of probes supported on particles is given.

Preferably, all the wells 16, 16 ··· are adjacent directly to the solution reservoir chamber 26, constituted by a space defined by the chip 10 and the vessel 12, through the filter 18 constituting the bottom wall 22 or the side wall 20.

For example, as shown in Figs. 12 (a) and 12 (b), this solution reservoir chamber 26 may be constituted by a space defined by the lower surface of the bottom of the chip 10 and the upper surface of the bottom of the vessel 12. Alternatively, as shown in Figs. 13 (a) and 13 (b), the solution reservoir chamber 26 may be constituted by a space defined by a chip 10 and a vessel 12 which are each independently provided.

According to the above construction, the solution resorvoired in the solution reservoir chamber 26 common to a plurality of wells 16, 16 ··· is stirred optionally under applied or reduced pressure to allow the solution to pass through a single-layer filter 18, and the solution then reaches, in a parallel manner, the plurality of wells 16, 16 ··· containing particles on which different types of probes are supported, resulting in contact and reaction in a parallel manner.

Further, the analyte remaining unreacted in a specific well 16 reaches the inside of other well 16 through the action of stirring or applied or reduced pressure, where contact and reaction are attempted. Thus, contact and reaction of the unreacted solution with the probe-supported particles are successively attempted in the wells 16, 16 ··· while conducting transfer of the solution among the wells 16, 16 ···.

Further, since the solution reservoir chamber 26 is adjacent to the wells 16, 16 ··· through the filter 18 having only a single layer, the pressure loss can be minimized. Further, since the arrival of the solution at the solution reservoir chamber 26 and the reaction of the solution with the probe-supported particles within the wells 16 are carried out in parallel, the contact and the reaction time can be minimized.

The pressure loss can be reduced to a very low level by setting the diameter and depth of pores in the filter 18 to suitable values, and the solution can be freely moved between all the wells 16, 16 ··· containing probe-supported particles and the solution reservoir chamber 26 constituting a common chamber for the solution, as if the solution is moved within one space free from the filter 18.

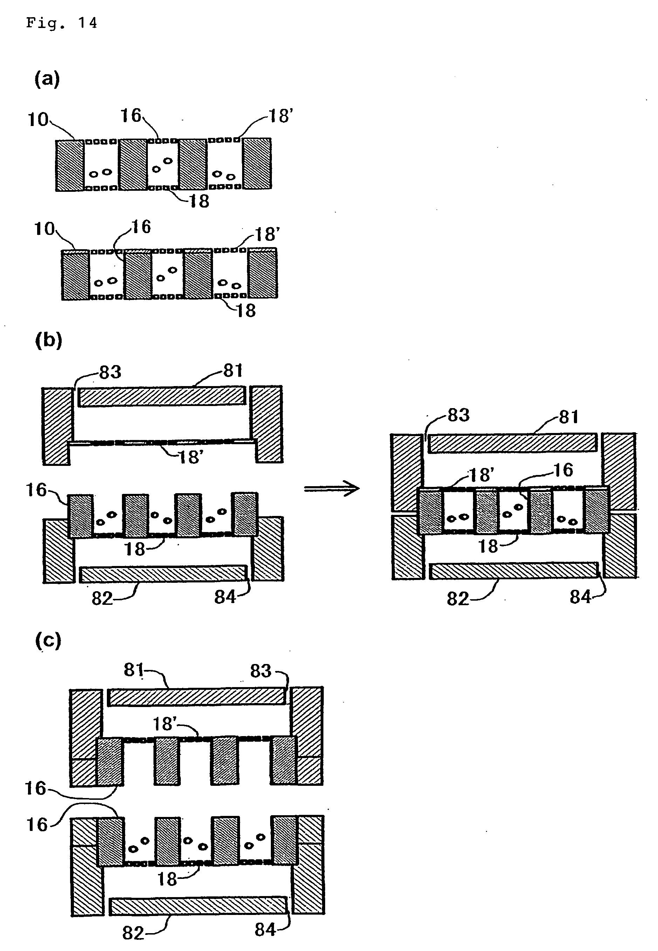

Alternatively, a construction may also be adopted in which a first filter is provided at the bottom part and a second filter is provided opposite to the first filter with the wells being interposed between the first and second filters. This embodiment is shown in Fig. 14 (a). As shown in the drawing, an upper filter 18' is provided as the second filter on the upper part of the biochip. This upper filter 18' is mounted after probe-supported particles are contained in the wells 16. Methods usable for mounting the upper filter 18' include those as described above, for example, adhesives, magnetic force, mechanical fitting, surface joining between smooth parts, and mechanical pressing. Further, a method may also be adopted in which, as shown in Fig. 14 (b), any of a filter 18' and a well 16 provided with a bottom filter 18 is previously mounted on each of an upper vessel 81 and a lower vessel 82, the upper vessel 81 is joined to the lower vessel 82 followed by mounting by a pressure-sensitive adhesive, a magnet, or mechanical pressure of a clamp or the like. In this method, the upper filter 18' and the bottom-side filter 18 can be arbitrarily separated from each other by setting/resetting of the mechanical pressure. Alternatively, a method may also be adopted in which, as shown in Fig. 14 (c), a well 16 provided with a bottom-face filter 18 is mounted on a lower vessel 82, a well 16 provided with a bottom-face filter 18 is mounted on an upper vessel 81 so that the assembly of the well 16 and the bottom-face filter 18 mounted on the upper vessel 81 and the assembly of the well 16 and the bottom-face filter 18 mounted on the lower vessel 82 are opposite to each other in the position of the top and the bottom of the assembly, and the upper vessel 81 and the lower vessel 82 are mounted by any of the above methods so that the well 16 mounted on the upper vessel 81 is jointed to the well 16 mounted on the lower vessel 82.

Further, the construction of the biochip kit may be as shown in Fig. 16 (a). Specifically, a smooth plate 87a with a through-hole having the same shape as the outer peripheral part of the well is stacked onto a plate 87b with a through-hole having a smaller diameter than the through-hole provided in the plate 87a, a chip is inserted into the level difference part of the hole, and, further, the assembly is covered with a transparent plate 94 having a liquid introduction/discharge hole so as to close the hole in the plate 87b. In this case, when the plates 87a, 87b are formed of, for example, a silicon wafer and the transparent plate 94 is formed of polished quartz glass, the kit can be formed by interface bonding utilizing smoothness without the use of any adhesive.

### <Method for operating biochip kit>

In the method for operating a biochip kit according to the present invention, in such a state that a solution with probe-supported particles dispersed therein contained in wells in the biochip can come into contact with various solutions contained in the vessel, for example, solutions containing analytes, various buffer solutions, washing solutions, separating media, labelling agents, secondary antibodies, sensitizers, proteases, ionizing agents or the like, the solution contained in the wells and the solution contained in the vessel are circulated, whereby both the solutions can be mixed, diffused, reacted, washed or separated with high efficiency.

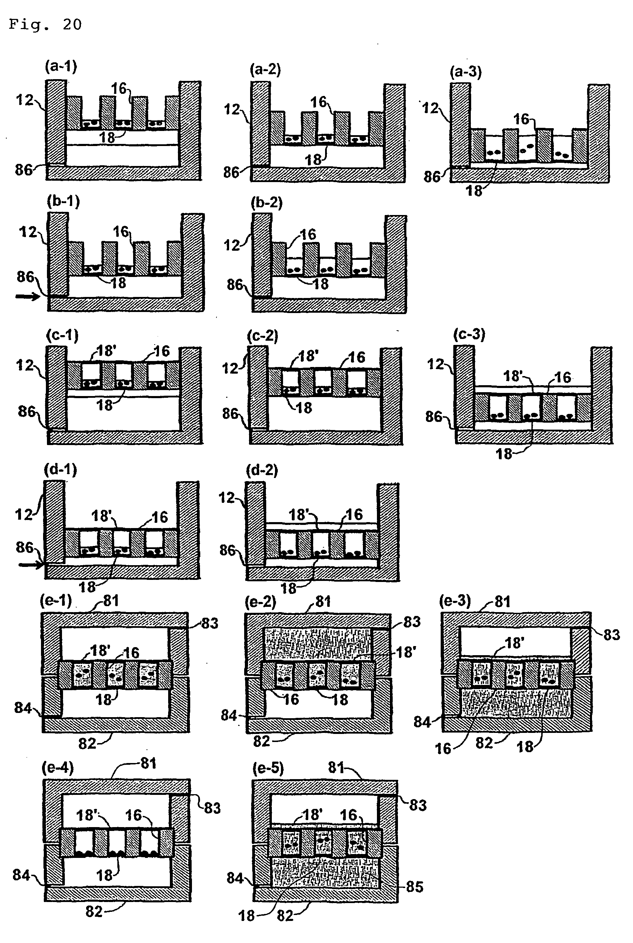

Methods usable for bringing both the solutions into contact with each other include a method in which the biochip is vertically moved within the solution contained in the vessel for contact with the solution contained in the vessel (Fig. 20 (a-1) → (a-3), Fig. 20 (c-1) → (c-3)), and a method in which the solution interface is vertically moved, for example, by bringing the solution within the vessel to a pressurized/depressurized state through a nozzle 86 or by externally introducing a solution into the vessel and discharging the solution from the vessel to transfer the solution to the wells to allow the solution from the vessel to come into contact with the solution within the wells (Fig. 20 (b-1) → (b-2), Fig. 20 (d-1) → (d-2)).

Thus, when the interface of the solution within the vessel is vertically moved by increasing or reducing the solution within the vessel, for example, by pressurization/depressurization or by external introduction of a solution and discharge of the solution from the vessel, in such a state that the probe-supported particles stay within the biochip, the solution contained in the wells is integrated with the solution contained in the vessel and, consequently, the solution contained in the wells can be moved to the outside the wells while the solution can be introduced from the outside of the wells into the well. Repetition of this procedure can realize high efficient mixing, diffusion, reaction, washing or separation of the solution contained in the vessel and the solution contained in the well.

Alternatively, the method as shown in Fig. 20 (e-1) to (e-5) may be carried out. Specifically, an upper vessel 81 provided with a filter 18' is joined to a lower vessel 82 provided with wells 16 having a filter 18 at their bottom part, and a nozzle 83 and a nozzle 84 for pressurization/depressurization and introduction/discharge of the solution are respectively provided on the upper vessel 81 and the lower vessel 82 (Fig. 20 (e-1)). Pressurization/depressurization is carried out through the nozzle 83 to allow the solution contained in the upper vessel 81 to come into contact with the solution contained in the wells 16, whereby the solution contained in the upper vessel 81 is mixed with the solution contained in the wells 16 (Fig. 20 (e-2)) and further is transferred to the lower vessel 82 (Fig. 20 (e-3)). The solution is then discharged to the outside of the vessel through the nozzle 84 by pressurization/depressurization (Fig. 20 (e-4)). Other solution 85 may also be filled into the wells (Fig. 20 (e-5)).

In this case, preferably, the volume of the upper vessel 81 is the same as the volume of the lower vessel 82, and the amount of the solution introduced is slightly larger than the volume of the upper and lower vessels, whereby, when the solution is transferred to the lower vessel 82, a part of the solution can stay in the upper vessel 81 and, thus, air is not bitten within the filter and the pressure necessary for the pressurization/depressurization can be minimized.

In the vertical movement of the wells and the movement of the solution, preferably, the movement is not continuously carried out but is intermittently carried out while providing predetermined movement stop times. Alternatively, preferably, during the movement in a positive direction, movement in a negative direction is interposed for a short period of time. According to this method, the particles contained in the wells do not stick to the filter and are circulated through within the wells by stop of the movement or by the movement in the negative direction, and, thus, the efficiency of contact between the solution contained in the biochip and the particles can be maximized.

### <Method for introducing analyte>

Analytes introduced into respective wells in the biochip may be the same or different. When an identical analyte is introduced into the wells, the identical analyte may be introduced by spotting with a spotter or the like from above the wells into the wells. Alternatively, a method may also be used in which the analyte is used as the solution contained in the vessel and this solution is brought into contact with the solution contained in the wells by any of the above methods to cause circulation, mixing, diffusion, and reaction between both the solutions.

When the number of wells in the chip is large, the method using an analyte within the vessel is preferred, because sequential spotting with a spotter requires a long spotting time and parallel spotting requires the use of a number of spotters.

On the other hand, when analytes introduced into respective wells are different from each other, different analytes are introduced from above the wells by spotting with spotters or the like into respective wells. Alternatively, a method may also be used in which different analytes contained in vessels provided independently for respective wells are brought into contact with the solutions contained in the wells by any of the above methods to cause circulation, mixing, diffusion, and reaction between the analytes and the solutions contained in the wells.

Analytes usable herein include, but are not limited to, various cultures, tissues, fungi, viruses, cells, lymphocytes, lipids, sugar chains, nucleic acids, urine, blood, serum, hemocytes, human/mouse cytokines, serine/threonine, kinase, proteins having a molecular weight of 50 to 1,000,000, that is, synthetic peptides, membrane proteins, enzymes, signal transduction proteins, transport proteins, phosphorylated proteins and other various proteins, cytokines, lymphokines, antibodies such as IgA and IgE, various antigens, transcription factors, low molecular lead compounds having a molecular weight of 50 to 1,000,000, substrates, coenzymes, regulators, lectins, hormones, neurotransmitters, antisense oligonucleotides, ribozymes, aptamers or other ligands, various drug candidate compounds, and various chemical compounds that have physiologically activity or have a possibility of having physiologically activity.

When competitive hybridization or competitive immunoassay reaction is used, a target-containing analyte and a standard-containing labelled analyte are simultaneously used in combination.

In addition to the analyte, different materials corresponding to the wells, for example, various buffer solutions, washing liquids, labelling agents, secondary antibodies, and sensitizers, may be introduced by any of the above methods.

### <Method for reacting target contained in analyte with probe>

In the method for reacting a target contained in an analyte with a probe using the biochip kit according to the present invention, the reaction is carried out by the following steps (1) to (3).
(1) The step of placing specific particles in wells in a biochip by any of the above methods. This step may be previously regulated by a chip manufacturer in the preparation of the chip.
(2) The step of introducing an analyte-containing solution into the wells in the biochip and bringing the analyte and the particles within all the wells to such a state that they come into contact with each other according to the above procedure. In this step, the solution contained in the vessel is integrated with the solution contained in the well to cause diffusion and reaction between the target contained in the analyte and the probe, for example, by vertically moving the wells within the solution contained in the vessel or by moving the interface of the solution contained in the vessel.
(3) The step of vertically moving the wells within the solution contained in the vessel in the biochip, or vertically moving the interface of the solution contained in the vessel in the biochip, to react the target contained in the analyte with the probe. In this step, the reaction of the analyte with the probe is allowed to proceed based on the above procedure.

### <B/F separation method>

The B/F separation method using the biochip kit according to the present invention is carried out by steps (1) to (3) in the above reaction method and the following steps (4) and (5).
(4) The step of lowering the height of the interface of the solution until the position of the interface of the solution is below the lower surface of the filter at the bottom of the wells to remove the analyte remaining unreacted with the probe supported on the particles from within each of the wells.
(5) The step of introducing a washing liquid into the wells in the biochip, circulating the washing liquid through the vessel into the wells in the biochip to introduce the washing liquid into the wells in the biochip and to discharge the washing liquid from the wells in the biochip, and discharging the washing liquid from the wells, whereby substances other than the probe-bound target are removed by washing.

### <Fractionation separation method>

The fractionation separation method for a target contained in an analyte using the biochip according to the present invention is carried out by steps (1) to (3) in the above reaction method and the following steps (4) to (6).
(4) The step of lowering the height of the interface of the solution until the position of the interface of the solution is below the lower surface of the filter at the bottom of the wells to remove the analyte remaining unreacted with the probe supported on the particles from within each of the wells.
(5) The step of introducing a washing liquid into the wells in the biochip, circulating the washing liquid through the vessel into the wells in the biochip to introduce the washing liquid into the wells in the biochip and to discharge the washing liquid from the wells in the biochip, whereby non-target substances other than the probe-bound target are removed.
(6) The step of fitting a concave part, a convex part, or a smooth part, provided on the lower end of the well side part of the biochip, and a convex part, a concave part, or a smooth part, corresponding to the concave part, convex part, or smooth part in the biochip, provided on the upper end of the vessel, together, and then introducing a separating agent solution into the wells in the biochip, whereby the target in the analyte is isolated from the particle and is transferred to the wells in the vessel.

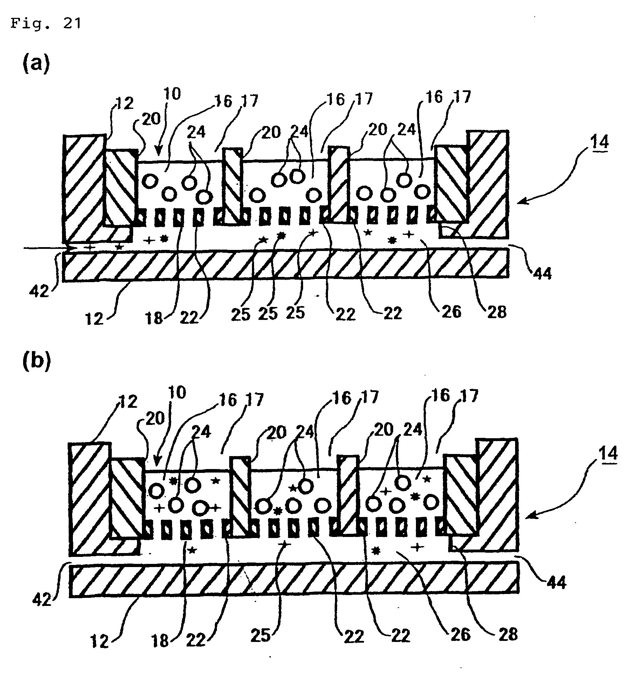

One embodiment of the fractionation separation method for an analyte according to the present invention will be specifically explained in conjunction with Figs. 21 to 23. At the outset, probe-supported particles different from each other in the type of probe supported on the particle are placed in respective wells 16 in a biochip. That is, probe-supported particles are placed in the wells so that the wells are different from each other in the type of probe supported on particles contained therein.

Next, as shown in Figs. 21 (a) and 21 (b), an analyte solution containing an analyte 25 is introduced into a vessel 12 in the biochip 10, and the analyte and the probe-supported particles 24 in all the wells 16 are brought to such a state that they can come into contact with each other. In this case, the height of the solution interface within the wells 16 should not exceed the height of the side walls 20.

The analyte-containing solution may be introduced to the predetermined interface height, for example, by the following methods. The first method is as follows. As in the biochip kit 14 shown in Fig. 13, a biochip kit comprising a chip 10 and a vessel 12 provided independently of each other is provided. An analyte solution is previously placed in the vessel 12. The chip 10 is moved downward and immsered in the analyte solution to introduce the analyte solution into the wells 16 through the filter 18. In this case, when there is a pressure loss in the filter 18, parts where the chip 10 and the vessel 12 are mutually contacted are sealed so that increased or reduced pressure can be applied to the filter 18, whereby the analyte solution can be passed through the filter 18 and lead to the inside of the wells 16.

The second method will be expalined. In the second method, as in the biochip kit 14 shown in Fig. 12, a chip in which the distance between the bottom face of the vessel 12 and the filter 18 is previously set to a given value is provided. An analyte solution is introduced from an introduction port formed in the lower wall or side wall in the vessel 12 through the solution reservoir chamber 26 into the wells 16.

The analyte solution may be introduced through an well opening 17. In this case, preferably, the analyte solution is introduced in an equal amount for each well 16 by means of a spotter or the like. When the analyte solution is introduced, for example, through an introduction port as indicated by a reference number 42 in Fig. 21, this introduction may be carried out, for example, by a method in which pressure is applied through the introduction port to feed the analyte solution into the vessel, or by a method in which the solution reservoir chamber 26 side is evacuated to feed the analyte solution into the vessel.

The analyte can be brought into contact with the probe-supported particles 24 contained in all the wells 16 by these methods (Fig. 21 (a)). In the method for conducting mixing, diffusion, and reaction between the probe-supported particles and the analyte solution in the wells 16, for example, as shown in Fig. 12 (b) and Fig. 13 (b), when the side walls 20 are formed of a filter 18 and adjacent wells are partitioned by the filter 18, the solution within the wells 16 can be replaced by agitating the analyte solution. The agitation can be carried out by conventional methods such as an agitator with an agitating blade, sonic or ultrasonic vibration, and agitation by movement of air or paramagnetic substances. Among them, sonic or ultrasonic vibration is particularly preferred. The sonic or ultrasonic vibration can be carried out by any of a method in which the vessel 12 or the chip 10 is vibrated and a method in which a vibrator is placed in the analyte solution contained in the chip 10. In this case, the frequency applied in the vibration may be properly regulated by the type of the probe and the analyte and may be 100 Hz to 1 GHz, preferably 1 kHz to 300 MHz. In this range of frequency, the impedance is low, and damage to the target can be minimized. When the applied frequency is less than 100 Hz, the agitation ability is low, while when the applied frequency exceeds 1 GHz, the analyte or the probe is likely to be damaged. Further, as shown in Fig. 13, when the vessel 12 and the chip 10 are provided independently of each other, after the chip 10 is pulled up from the interface of the solution contained in the vessel 12, the solution contained in the vessel 12 is agitated by any of the above methods and the solution contained in the wells 16 can be replaced by any of a method in which the chip 10 is again immersed in the solution contained in the vessel 12 and a method in which the chip 10 is horizontally rotated or vertically moved to relatively move the vessel 12 and the chip 10. When the wells 16 are partitioned by side walls which are not the filter 18, the solution can be replaced by leading the solution to the solution reservoir chamber 26 through the filter 18 in the bottom wall 22 and then leading the solution to other wells 16 through the filter 18.

Alternatively, also in the case of any of the chips shown in Figs. 12 and 13, the analyte solution can be introduced into the wells 16 by introducing pressurized air through an introduction hole 42 to increase the interface of the analyte solution contained in the vessel 12.

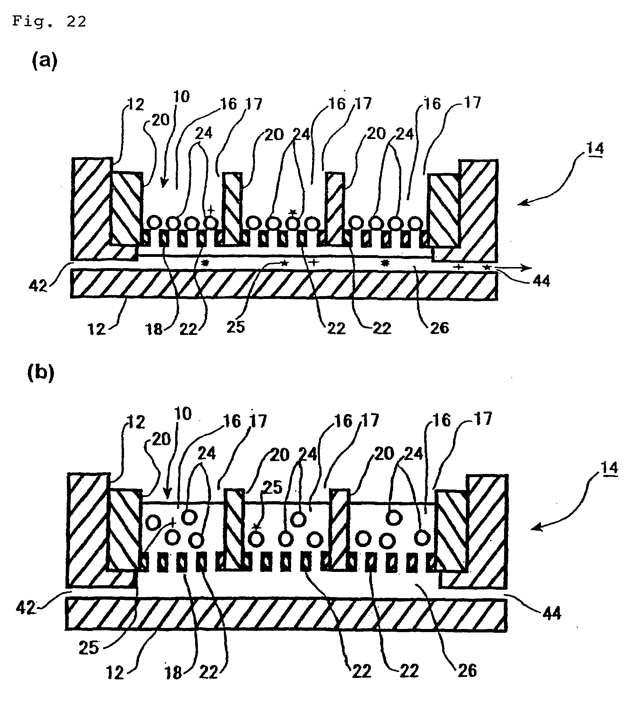

Next, as shown in Fig. 22 (a), the height of the interface of the analyte solution is lowered to a position below the lower surface of the bottom wall 22 of the wells 16 to remove non-target substances remaining unreacted with the probe in the probe-supported particles 24. The height of the solution interface may be lowered to a level below the bottom face of the filter 18 by a method in which the analyte solution is transferred through the filter 18 provided on the bottom wall 22 from a discharge port 44 to the outside of the vessel 12 or transferred to the solution reservoir chamber 26, for example, by a pump or increased pressure or reduced pressure. When the vessel 12 and the chip 10 are provided independently of each other, methods usable herein include a method in which the vessel 12 and the chip 10 are relatively moved to vertically move the filter 18 and a method in which the chip 10 is transferred to the vessel adjacent to the biochip 10 or is transferred to an independent separated vessel.

In the above embodiment, wells with one side thereof being open are used. In the case of a chip as shown in Fig. 14 in which filters face each other, there is no fear of leakage of particles from the wells. Further, this case, the liquid level can be moved as desired beyond the height of the wells.

Further, before and/or after this operation, if necessary, various detergents and various buffer solutions or liquid chemicals such as secondary antibodies may be introduced. They may be introduced in the same manner as in the introduction of the analyte.

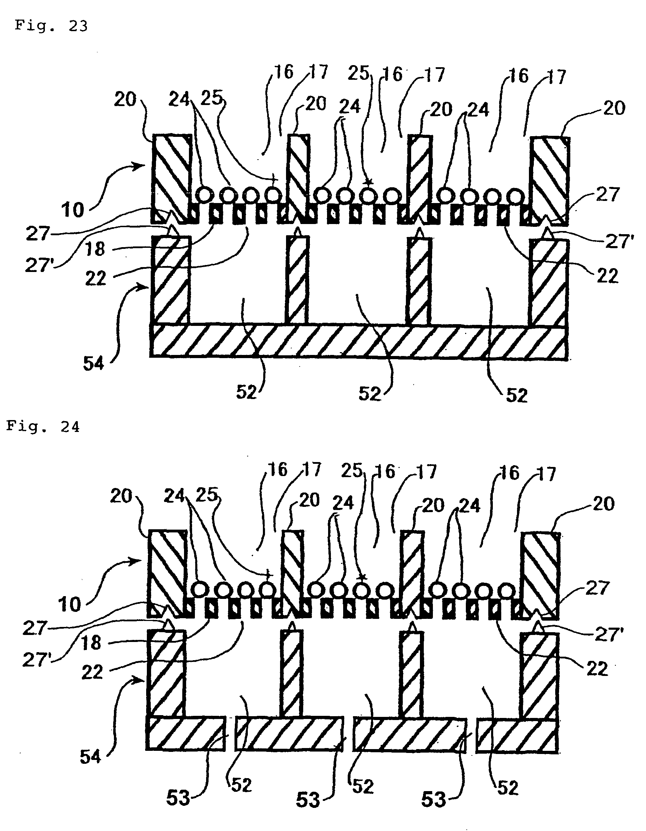

Next, as shown in Fig. 23, a vessel 54 with a plurality of reservoir wells 52 corresponding to respective wells 16 is provided. A separating agent solution is introduced from the upper openings 17 into the wells 16 to separate, from the probe-supported particles reacted with a target contained in the analyte, a target which is then transferred to the vessel 54.

As shown in Fig. 24, this vessel 54 may be a vessel having minute through-holes 53 at the bottom face of the wells 52. When the through-holes are provided, a differential pressure can be created between the chip wells 16 and the vessel wells 52. Consequently, the liquids contained in the wells 16 in the chip can be transferred at a time by flow-down movement. According to the simultaneous flow-down movement, the number of times of transfer necessary for the transfer of the liquids from the chip wells to the vessel wells is advantageously smaller than that in the method in which the transfer is carried out in the number of times which is identical to the number of wells through a syringe or the like. Further, contamination, for example, through the syringe or a reduction in the amount of the analyte due to the deposition of the analyte to the syringe can be prevented.

The diameter of the through-holes formed on the bottom face of the vessel is preferably 100 to 500 µm, more preferably 150 to 300 µm, although the diameter also depends upon the height of the holes. When the hole diameter exceeds 500 µm, there is a fear of flowout of the liquid from the bottom face. On the other hand, when the hole diameter is less than 100 µm, the resistance at the time of pressurization/depressurization is disadvantageously increased.

The separating agent solution can be introduced by means of a spotter or the like through the well opening 17, either individually for respective wells 16 or at a time into the wells 16.

The vessel 54 to which the solution is transferred after target separation is not particularly limited so far as the vessel 54 is provided with a plurality of target reservoir wells 52, 52 ··· corresponding to the respective wells 16. For example, a method may be adopted in which a vessel 54 provided with target reservoir wells 52, 52 ··· having sizes substantially identical to the wells 16, 16 ··· is located right under the chip 10 and a separating agent solution is introduced through the well openings 17 to separate, from the probe-supported particles contained in the wells 16, the target which is then placed in the target reservoir wells 52 located right under the wells 16.

Also in the construction shown in Figs. 14 (a) and (b) and (c) in which a filter is provided at both the bottom part and the upper part of the wells, the operation can be carried out according to the steps described above. For example, in the case of a chip shown in Fig. 14 (b), as shown in Figs. 20 (e-2) and (e-3), increased or reduced pressure air can be introduced or discharged through nozzles 83, 84 to integrate the solution contained in the upper vessel 81 with the solution contained in the wells 16 and thus to gradually transfer the solution to the upper vessel 81/lower vessel 82.

The analyte which has been separated and fractionated by the above method can be used as a starting material for the next reaction step or as a purified product for assay. In particular, the separated and fractionated analyte can be introduced into a detection apparatus such as a mass spectrometer, a Raman spectrometer, a surface plasma spectrometer, an X-ray spectrometer, an electrochemical detector, and a quartz crystal microbalance detector in which the analyte can be analyzed and identified in detail by an analytical method that does not require the use of the so-called labelling agent.

In the analysis by these means, the so-called kinetic analysis can be carried out by varying reaction conditions such as temperature or by conducting detection with the elapse of time a plurality of times. Further, the concentration or amount of the target contained in the analyte can be determined by analyzing not only details of the analyte and the presence of a ligand reacted with the probe but also details of each particle within the wells 16.

### <Analyte detection method>

In the method for optically detecting and identifying the target contained in the analyte according to the present invention, after the same step as the above method for reacting the target contained in the analyte with the probe, or the B/F separation method is carried out, the following steps (1) to (3) are carried out.
(1) A labelling agent is introduced to bind the labelling agent to the probe and the target, and the labelling agent remaining unbonded is then washed away.
(2) The solution contained in the wells is passed through the filter at the bottom part of the wells to discharge the solution from within the wells to the outside of the wells and to position the particles contained in the wells on the pores in the filter.
(3) A reaction or interaction between the probe supported on the particles with the target contained in the analyte is detected.

In the case of the so-called homogeneous assay method, the B/F separation step may be omitted.

Labelling agents usable in step (1) include, for example, radioisotopes, organic fluophors, rare earth or other complex fluophors, fluorescent proteins, phosphors, luminescent nanoparticles utilizing quantum efficiency, chemiluminescent agents, enzyme luminescent agents, and nanoparticles of gold and silver.

Further, for example, secondary probes such as secondary antibodies with the above labelling agent bound thereto, fluorescent molecules/quenching molecules for a molecular beacon method, donor and acceptor molecules for a FRET (fluorescent resonance energy transfer) method may also be used.

In step (3), for each particle, both probe identification information of the particle and information about a reaction or interaction between the probe supported on the particle and the target contained in the analyte can be detected. Further, for particles in each well, it is possible that information about identification of the probe supported on the particle is identified, and the state of an interaction between the probe and the target contained in the analyte is then measured to calculate information about an interaction for each well based on information about the state of interaction obtained for each particle.

One embodiment of the method for detecting an analyte according to the present invention will be described in detail. At the outset, particles with different probes supported thereon are placed in respective wells. That is, probe-supported particles are placed in the well so that the well are different from each other in the type of probe supported on the particle contained therein. When the biochip according to the present invention is used in the detection of an analyte, the number of particles for each probe in each well is preferably 1 to 1000, more preferably 1 to 500, still more preferably 1 to 200.

Next, an analyte-containing solution is introduced into the vessel 12 in the biochip 10 to allow the analyte to come into contact with the probe-supported particles within the wells 16 (Figs. 21 (a) and (b)).

The height of the interface of the analyte solution is then lowered to a position below the lower surface of the filter 18 in the wells 16 to remove the analyte remaining unreacted with the probe supported on the probe-supported particle 24 (Fig. 22 (a)).

These steps are carried out according to the procedure used in the above-described method for separating and fractionating an analyte. In the step of removing the analyte remaining unreacted, as shown in Fig. 22 (b), a method may be adopted in which a washing liquid is introduced to wash the particles and, if necessary, introduction, agitation, and discharge of the washing liquid are repeated to remove the analyte remaining unreacted from the probe-supported particles.

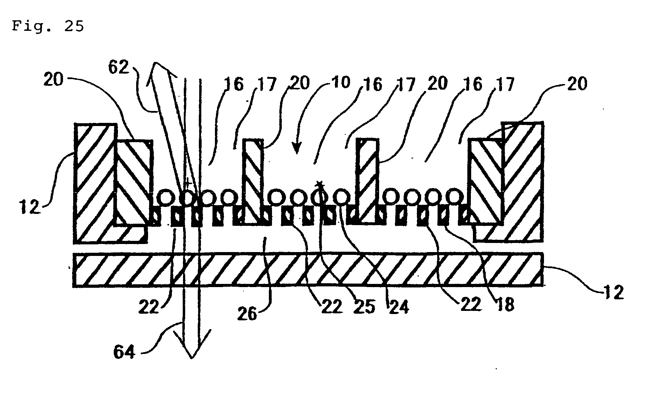

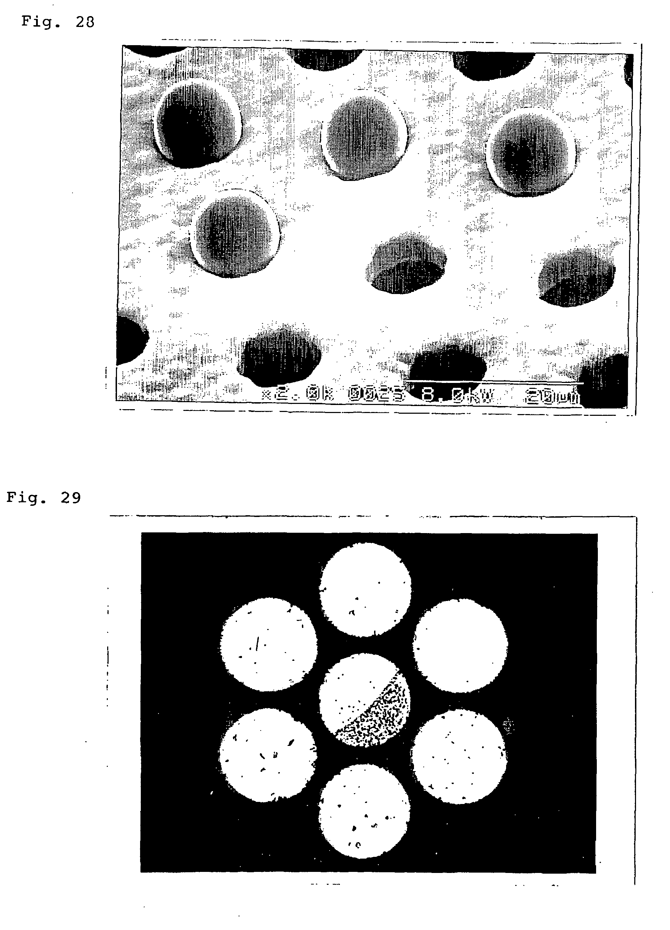

Next, as shown in Fig. 25, the solution contained in the biochip is discharged through the bottom-side filter to position the particles in the wells 16 on the pore part in the bottom-side filter 18, and, thereafter, for each well 16, a reaction or interaction between the probe-supported particles and the analyte is detected. Fig. 28 shows an electron photomicrograph of such a state that probe-supported particles are actually positioned on filter pores.

In the prior art technique, when a reaction of the probe-supported particle with the analyte is detected within the solution, a part or the whole of the particles contributed to the reaction is detected and identified as one analyte. By contrast, when the height of the interface of the solution is lowered to the position below the lower surface of the filter 18 and detection is carried out in such a state that the probe-supported particles are located on filter pores, all the particles contributed to the reaction can be detected and identified for each particle, contributing to improved detection sensitivity. Fig. 28 shows an electron photomicrograph of probe-supported particles located on filter pores.

The analyte can be detected by various conventional methods, and examples of detection methods usable herein include, but are not limited to, radioisotope detection, fluorescent detection, chemiluminescent detection, enzyme luminescent detection, and Raman detection.

In the analysis by these methods, the so-called kinetic analysis can be carried out by varying temperature or by conducting detection with the elapse of time a plurality of times. Further, the concentration or amount of the ligand contained in the analyte can be determined by analyzing not only details of the analyte and the presence of a ligand reacted with the probe but also details of each particle within the wells.

Further, in the so-called optical detection such as fluorescent detection or chemiluminescent detection, preferably, the chip is taken out of a vessel for housing the chip comprising wells, and the chip independently undergoes irradiation with excitation light and detection of detection light. When the chip is taken out of the vessel, the excitation light can be applied to any of the upper surface and lower surface of the chip. Further, the detection light can be detected from any of the upper surface and lower surface of the chip.

Since the chip is taken out of the vessel, deformation of the vessel and the influence of the vessel- derived autofluorescence can be eliminated. Further, since distance to the object is reduced, the numerical aperture NA can be increased in the optical detection.

As shown in Fig. 16, when the vessel is transparent, any of excitation light and detection light can be taken out from the top and bottom of the chip without the need to take the chip out of the vessel. In this case, the NA value can be increased by minimizing the thickness of the transparent plate 94 shown in Fig. 16 and the thickness of the plate 87b.

In the chip shown in Figs. 14 (b) and 14 (c), a method may also be adopted in which, for detection after the reaction, the upper vessel 81 with an upper filter is removed and, for the lower vessel 82 with a lower filter on which the particles are arranged, light is applied from the upper open face for detection.

Explanation will be given about the case where, in the above detection step, for each well, the state of interaction between the probe supported on the particle and the analyte is measured and information about the interaction on a well basis is calculated based on the information about the state of interaction obtained for each particle. At the outset, for each probe-supported particle contained in each well, information about interaction between the probe and the analyte is measured on a well basis. The above-described various methods can be used as the measuring means. For example, in the case of fluorescent detection, excitation light such as UV light or visible light for detection is applied to the particles arranged within an identical well, and a signal such as detection light obtained upon the application of the excitation light is detected, for example, by a CCD camera or a fluorescent tube. This method is carried out for each individual particle and the detection procedure is likewise successively carried out for particles contained in other wells. Next, the data thus obtained are subjected to cumulative processing for each well, and statistical standardization is carried out for each data on number of particles N contained in the well. If necessary, the data thus obtained can be compared with existing data in various databases for correction or judgment of the data.

Unlike, for example, the optical detection method on a well or cell basis in the conventional biochip in which fluorescence intensity and fluorescence spectrum for one well or cell are measured, according to the above method, information for the number of particle probes N within each well is obtained, and, thus, the amount of information is so large that the sensitivity is improved. When the distribution of information for N particle probes is statistically processed, quantitative detection and identification can be carried out.

In the so-called kinetic analysis in which reaction conditions such as temperature are varied, or the detection is carried out with the elapse of time a plurality of times, when, for each particle within the well, the detection or the particle detection pattern is traced with the elapse of time, analytical data which are large in population parameters N and are highly reliable can be obtained.

In the present invention, biochips include DNA chips comprising nucleic acids as a probe supported on particles and, in addition, protein chips comprising proteins such as antigens and antibodies or chemical substances reactive with these proteins supported on particles, chips in which low molecular compounds are supported and interaction with proteins or the like is screened, sugar chain chips comprising sugar chains supported on particles, and cell chips comprising cells supported on particles.

These DNA chips, protein chips, sugar chain chips, and cell chips can be applied to, depending upon various probes immobilized on the carrier, gene function analyses; gene expression analyses; functional proteomics or structural proteomics; functional selloum, structural selloum, disease analyses, and clinical tests for confirming affected conditions for diseases such as various infectious diseases; detection of gene polytypes; selection of therapeutic drugs according to the patient's gene sequence, called tailor-made medical treatment or chemotherapy; research for interaction between nucleic acids, proteins, cells, or sugar chains and chemical compounds, and search for orphan receptor ligands; pharmacological screening or safety and toxicity screening for chemical compounds for development of drugs, called toxicogenomics; and other various types of screening and the like. Further, combining specific analytes with specific probes can realize various assay systems, for example, interaction assays such as immunoassay, protein-DNA, and protein-protein, protein, sugar chain, cell-ligand assay, receptor-ligand assay, and enzyme assay such as kinase.

The present invention will be descried with reference to the following examples. However, it should be noted that the present invention is not limited to these Examples only.

### [Example 1]

### Preparation of filter and rib by electroforming

### 1. Preparation of filter

A resist THB-110N (tradename: manufactured by JSR Corporation) was spin coated onto a stainless steel substrate (SUS 304, dimension 120 mm x 120 mm x 1 mm in thickness) to a thickness of 5 µm, and the coating was prebaked on a hot plate at 120°C for 5 min. After the prebaking, the coating was exposed in a predetermined pattern form using a mask aligner M-3LDF (tradename: manufactured by Mikasa Corp.) at an exposure of 400 mJ/cm² so that non-electroplated parts remain unremoved. Development was carried out with a developing solution PD523 (tradename: manufactured by JSR Corporation), followed by post-baking on a hot plate at 90°C for 5 min.

Next, the SUS substrate which had been patterned using the resist THB-110N was placed in a nickel sulfamate bath (composition of bath: 700 g/liter 60% nickel sulfamate solution, 5 g/liter nickel bromide, 35 g/liter boric acid, 1.5 g/liter stress regulator, and 2.5 ml/liter pit preventive agent) maintained at pH 4.0 to 4.5 and bath temperature 50°C. The voltage and current were regulated so that the current density was 1 A/dm² at a voltage of 7 V. Under these conditions, direct current was applied for 30 min to form a nickel film having a thickness of 5 µm, a minimum pore diameter of 3 µm, and a minimum pore spacing of 7 µm.

After the electroplating, the electroforming sample was immersed in a resist stripper THB-S1 (tradename: manufactured by JSR Corporation) with stirring for 30 min to peel off the whole resist to prepare a filter.

### 2. Preparation of rib

The above procedure was repeated to stack a rib onto the nickel filter prepared by the electroforming, thereby preparing a filter with a rib. The rib was formed under substantially the same apparatus and electroforming conditions as those used in the preparation of the filter, except that the thickness of the rib was 50 µm (filter thickness: 5 µm). In order to form the rib having this thickness, a resist THB-220N (tradename: manufactured by JSR Corporation) was spin coated, and the coating was exposed at an exposure of 650 mJ/cm². Further, nickel plating was carried out for 5 hr to form a 50 µm-thick nickel plating film.

### [Example 2]

### Preparation of filter and rib by etching of silicon wafer with oxide film

### 1. Preparation of filter

A resist IX1170G (tradename: manufactured by JSR Corporation) was spin coated using a spinner (CLEAN TRACK MARK 8 (tradename): manufactured by Tokyo Electron Limited) on a 6-in. silicon wafer (thickness: 2 µm) with an oxide film at 3300 rpm for 30 sec. The coating was dried on a hot plate (CLEAN TRACK MARK 8 (tradename: manufactured by Tokyo Electron Limited)) at 90°C for 60 sec to form a 0.86 µm-thick resist film. This resist film was exposed using a reduced projection exposure system NSR-2205i12D (tradename: manufactured by NIKON CORPORATION, NA = 0.57, sigma = 0.60) at an exposure of 0.4 umC/H 1000 msec and 0.8 umC/H 580 msec, and paddle development was then carried out with a developing solution PD523AD (tradename: manufactured by JSR Corporation) at 23°C for one min. Next, water washing was carried out for 20 sec, followed by drying to form a resist pattern.

CF₄ plasma was applied in an RIE mode to the resist-patterned 2 µm-thick silicon wafer with an oxide film using a plasma etching apparatus. The resist was then removed with O₂ plasma to prepare a filter in which filter pores having a height of 2 µm and the following pore diameter and pore spacing had been formed in the oxide film: 0.4/0.8 µm, 0.8/0.8 µm, 2.8/1.0 µm, 2.8/2.0 µm, 2.8/2.8 µm, 2.8/4.0 µm, 4.0/1.0 µm, 4.0/2.0 µm, 4.0/4.0 µm, 4.0/6.0 µm, 4.0/8.0 µm, 8.0/2.0 µm, 8.0/4.0 µm, and 8.0/6.0 µm.

### 2. Preparation of rib

A resist (THB-151N) was spin coated with a spinner (spin coater 1H-DX2 (tradename): manufactured by Mikasa Corp.) at 1700 rpm for 20 sec on the filter in its side remote from the treated side. The coating was dried on a hot plate at 120°C for 5 min to form a 40 µm-thick resist film. This resist film was exposed using a one-to-one projection aligner MA-150 CC (tradename: manufactured by SUSS MicroTec KK), and paddle development was then carried out with a developing solution PD523AD (tradename: manufactured by JSR Corporation) at 23°C for 90 sec. Subsequently, water washing was carried out for 30 sec, followed by drying to form a resist pattern.

SF₆ plasma was applied in an RIE mode to the resist-patterned 440 µm-thick silicon wafer with an oxide film using a plasma etching apparatus. The resist was then removed with O₂ plasma to prepare a silicon wafer with wells having a hole having a height of 440 µm and a diameter of 500 µm formed in the silicon. This was cut with a dicing saw to prepare a chip with 28 wells in 10 mm square.

### [Example 3]

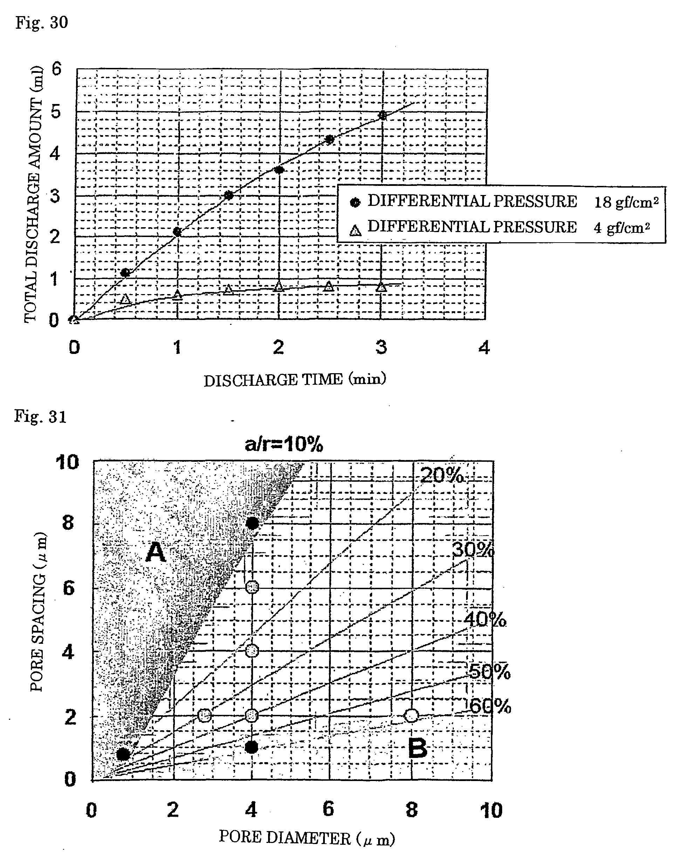

A solution prepared by diluting bovine serum with PBS (phosphate buffered saline) by 10 times was provided. The chip prepared in Example 2 having a filter pore diameter of 4.0 µm and a pore spacing of 2.0 µm was provided. The chip was set in the vessel shown in Fig. 16 (b). A tube was set in the upper lid, and a tank containing the diluted solution of bovine serum was connected to the other end face of the tube. The height of the tank was set so that a differential pressure of 4 gf/cm² and 18 gf/cm² could be provided. The diluted solution of bovine serum was flowed to the filter part in the chip vessel, and the diluted solution of bovine serum was discharged from the lower lid in the lower vessel. The relationship between the total discharge amount and the discharge time is shown in Fig. 30.

As shown in the drawing, the amount of discharge from the filter was substantially constant, and, during the evaluation period, neither clogging nor breaking occurred.

### [Example 4]

The same test as in Example 3 was carried out, except that eight combinations of chip pore diameter and pore spacing, which are different from those in Example 3, were used and the differential pressure was 18 gf/cm². The results are shown in Fig. 31. In this drawing, a/r represents the numerical aperture of the filter. As a result, it was found that, in Fig. 31, in region A (numerical aperture: less than 10%), the filter was likely to be clogged, and, in region B (numerical aperture: more than 60%), the filter was broken. Thus, when the numerical aperture was 10% to 60%, neither clogging nor breaking occurred.

## Claims

1. A biochip **characterized by** comprising a well(s) having, at its bottom, a filter comprising straight pores with a uniform diameter arranged at uniform pore spacings.

2. The biochip according to claim 1, **characterized in that** said filter has a thickness of 1 to 10 µm.

3. The biochip according to claim 1 or 2, **characterized in that** the open area ratio of the filter is 15 to 60%.

4. The biochip according to any one of claims 1 to 3, **characterized in that** the surface of the filter is formed of silica, titania, or alumina.

5. The biochip according to any one of claims 1 to 4, **characterized by** comprising a plurality of said wells provided integrally with each other.

6. The biochip according to any one of claims 1 to 4, **characterized in that** said well is singularly provided.

7. The biochip according to any one of claims 1 to 6, **characterized in that** a reinforcing rib part is provided on the upper side or lower side of said filter in said well.

8. The biochip according to claim 7, **characterized in that** said reinforcing rib part is of an integral type provided with a plurality of through-holes.

9. The biochip according to claim 7 or 8, **characterized in that** said reinforcing rib part is joined directly to said filter.

10. The biochip according to claim 7 or 8, **characterized in that** said reinforcing rib part is formed so as to continuously extend from said filter, said reinforcing rib part and said filter being formed of an identical material.

11. The biochip according to any one of claims 1 to 10, **characterized in that** a nonporous part free from pores of said filter is provided on the bottom of said well in a predetermined width from the periphery of said well.

12. The biochip according to any one of claims 1 to 11, **characterized in that** a first filter is provided at the bottom of the well(s) and a second filter is provided on the side opposite to the first filter so that the well(s) is sandwiched between said first and second filters.

13. The biochip according to any one of claims 1 to 12, **characterized in that** a dispersion with probe-supported particles dispersed therein is placed in said well(s).

14. The biochip according to claim 13, **characterized in that** the ratio between the diameter of said particles and the pore diameter of said filter is particle diameter/pore diameter = 1.1 to 2.5, and said particle diameter and said pore spacing satisfy a relationship represented by formula: particle diameter < pore spacing < particle diameter x 10.

15. The biochip according to claim 13, **characterized in that** the diameter of said particle and the pore diameter and pore spacing of said filter satisfy a relationship represented by formula: particle diameter > pore diameter + pore spacing/2.

16. The biochip according to claim 13, **characterized in that** said well contains a dispersion in which probe-supported particles having at least one identification means for providing probe identification information has been dispersed.

17. The biochip according to claim 16, **characterized in that** said identification means is at least one means selected from color, shape, diameter and gene sequence in said probe-supported particle.

18. The biochip according to claim 16 or 17, **characterized in that** a plurality of probe-supported particles which are identical to each other in probe identification information in all of said identification means are contained in an identical well and said wells are identical to each other in said probe identification information for a plurality of probe-supported particles contained therein.

19. The biochip according to claim 16 or 17, **characterized in that** a plurality of probe-supported particles which are identical to each other in probe identification information in all of said identification means are contained in an identical well and said wells are different from each other in said probe identification information for a plurality of probe-supported particles contained therein.

20. The biochip according to claim 16 or 17, **characterized in that** a plurality of probe-supported particles which are different from each other in probe identification information in said at least one identification means are contained in an identical well and said wells are identical to each other in construction of said probe identification information in all the identification means for a plurality of probe-supported particles contained therein.

21. The biochip according to claim 16 or 17, **characterized in that** a plurality of probe-supported particles which are different from each other in probe identification information in said at least one identification means are contained in an identical well and said wells are different from each other in construction of said probe identification information in at least one of said identification means for a plurality of probe-supported particles contained therein.

22. A biochip kit **characterized by** comprising: a vessel; and a plurality of wells formed integrally with each other or a single well in the biochip according to any of claims 1 to 21 housed in or connected to said vessel.

23. The biochip kit according to claim 22, **characterized in that** said vessel is formed integrally with said well(s).

24. The biochip kit according to claim 22, **characterized in that** said vessel is formed independently of said well(s).

25. The biochip kit according to any of claims 22 to 24, **characterized in that** said vessel is provided with well(s) corresponding to said well(s) in said biochip.

26. The biochip kit according to claim 25, **characterized in that** a through-hole is provided at the bottom of said well(s) in said vessel.

27. The biochip kit according to claim 25 or 26, **characterized in that** said biochip and said vessel are connected to each other so that the corresponding wells are connected to each other.

28. The biochip kit according to any of claims 22 to 27, **characterized in that** said vessel comprises a plurality of plates stacked on top of each other, said plurality of plates being each selected from plates with a through-hole and plates free from a through-hole.

29. A biochip kit **characterized by** comprising a plurality of biochips according to any of claims 1 to 21 which are connected to each other so that the corresponding wells are connected to each other.

30. The biochip kit according to any of claims 22 to 29, **characterized in that** the flat part provided on the lower end of the well side part in said biochip is connected directly to the flat part provided on the upper end of the well side part in said separate vessel or said separate biochip so that the wells are connected to each other.

31. The biochip kit according to any of claims 22 to 29, **characterized in that** either a positioning concave part into which a convex part provided on the upper end of the well side part in said separate vessel or said separate biochip is to be fitted, or a positioning convex part into which a concave part provided on the upper end of the well side part in said separate vessel or said separate biochip is to be fitted is provided on the lower end of the well side part in said biochip.

32. A process for producing a biochip according to any of claims 1 to 21, **characterized by** comprising: providing a plate having a structure of at least two layers different from each other in composition of a material constituting the layer; subjecting said plate to pattern etching from its one side to the boundary between the two layers to form a well hole(s); and subjecting said plate to pattern etching from its other side to the boundary between the two layers to form filter pores, thereby preparing a biochip comprising a well(s) and a filter connected to each other.

33. A process for producing a biochip according to any of claims 1 to 21, **characterized in that** silicon wafers are etched to prepare a filter, a rib, and a well which are then stacked on top of each other.

34. A method for operating a biochip kit, **characterized in that**, in a biochip kit according to any of claims 22 to 31 comprising said vessel and said biochip, said vessel being provided independently of said well(s) in said biochip, a solution is placed in said vessel and said well(s) in said biochip is vertically moved in said solution to bring said solution in said vessel into contact with said probe-supported particles and/or solution within said well(s).

35. A method for operating a biochip kit, **characterized in that** the interface of a solution contained in said vessel in a biochip kit according to any of claims 22 to 31 is vertically moved to bring said solution in said vessel into contact with said probe-supported particles and/or solution within said well(s).

36. A method for operating a biochip kit, **characterized in that**, in a biochip kit according to any of claims 22 to 31 comprising said vessel for housing said biochip therein, a pressure differential is created between said vessel and said chip or between mutual wells in said chip to cause contact of a liquid with said probe-supported particle within said well, transfer of a liquid between wells, or both of them.

37. A method for operating a biochip kit, **characterized in that**, in a biochip kit according to any of claims 22 to 31 comprising said vessel connected to said biochip, a pressure differential is created between said vessel and said chip or between mutual wells in said chip to cause contact of a liquid with said probe-supported particles within said well(s), transfer of a liquid between wells, or both of them.

38. The method for operating a biochip kit according to any of claims 34 to 37, **characterized in that** the solution within said vessel is brought into contact with said probe-supported particles and/or solution within said well(s) to perform mixing, diffusion, reaction, separation, or washing of contents within said biochip.

39. The method for operating a biochip kit according to any of claims 34 to 38, **characterized in that** an identical analyte is introduced into each well in said biochip.

40. The method for operating a biochip kit according to any of claims 34 to 38, **characterized in that** analytes introduced into respective wells in said biochip are different from each other.

41. A method for reacting a target contained in an analyte with a probe, **characterized by** comprising the steps of:
placing specific particles in said wells of said biochip in a kit according to any of claims 22 to 31 to constitute a chip according to any of claims 18 to 21;
introducing an analyte-containing solution into said wells in said biochip to bring the system to such a state that said analyte can come into contact with said particles within all the wells; and
vertically moving said wells within said solution contained in the vessel of said biochip, or vertically moving the interface of said solution contained in the vessel of said biochip, or applying a differential pressure to circulate said solution present within or outside said wells to react said target contained in said analyte with said probe.

42. A method for B/F separation of a target from an analyte, **characterized by** comprising the steps of:
placing specific particles in said wells in said biochip in a kit according to any of claims 22 to 31 to constitute a chip according to any of claims 18 to 21;
introducing an analyte-containing solution into said wells of said biochip to bring the system to such a state that said analyte can come into contact with said particles within all the wells;
lowering the height of the interface of said solution until the position of the interface of said solution is below the lower surface of said filter at the bottom of said well to remove said analyte remaining unreacted with the probe supported on the particle from within each of said wells; and
introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing.

43. A method for fractionally isolating a target in an analyte, **characterized by** comprising the steps of:
placing specific particles in said wells of said biochip in a kit according to claim 30 or 31 to constitute a chip according to any of claims 18 to 21;
introducing an analyte-containing solution into said wells of said biochip to bring the system to such a state that said analyte can come into contact with said particles within all the wells;
lowering the height of the interface of said solution until the position of the interface of said solution is below the lower surface of said filter at the bottom of said well to remove said analyte remaining unreacted with the probe supported on the particle from within each of said wells;
introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing; and
fitting a concave part, a convex part, or a smooth part, provided on the lower end of the well side part of said biochip, and a convex part, a concave part, or a smooth part, corresponding to the concave part, convex part, or smooth part in said biochip, provided on the upper end of said vessel, together, and then introducing a separating agent solution into said wells of said biochip, whereby said target in said analyte is isolated from said particle and is transferred to said wells of said vessel.

44. A method for detecting and identifying an interaction between a target contained in an analyte and a probe, **characterized by** comprising the steps of:
placing specific particles in said wells of said biochip in a kit according to any of claims 22 to 31 to constitute a chip according to any of claims 18 to 21;
introducing an analyte-containing solution into said wells of said biochip to bring the system to such a state that said analyte can come into contact with said particles within all the wells;
lowering the height of the interface of said solution until the position of the interface of said solution is below the lower surface of said filter at the bottom of said well to remove said analyte remaining unreacted with the probe supported on the particle from within each of said wells;
introducing a washing liquid into said wells in said biochip, circulating said washing liquid through said vessel into said wells in said biochip to introduce said washing liquid into said wells in said biochip and discharge said washing liquid from said wells in said biochip, and discharging said washing liquid from said wells, whereby substances other than the probe-bound target are removed by washing;
positioning said particles within said wells on said pores in said filter; and
detecting and identifying a reaction or an interaction between the probe supported on said particles and the target in said analyte.

45. The method for detecting and identifying a target contained in an analyte according to claim 44, **characterized in that**, for each particle, both probe indentification information of said particle and information about a reaction or interaction between said probe supported on said particle and said target contained in said analyte are detected.

46. A method for detecting and identifying a target contained in an analyte according to claim 44, **characterized in that**, for said particles in each well, information about identification of said probe supported on said particles is identified, and the state of an interaction between said probe and said target contained in said analyte is then measured to calculate information about an interaction for each well based on information about the state of interaction for each particle.
